# EUROPEAN PATENT APPLICATION

(11) **EP 4 814 120 A1**
(43) Date of publication of application: **30.09.2026**
(21) Application number: 25895235.7
(22) Date of filing: 12.08.2025
(51) Int. Cl.: C12N 15/77, C12N 9/88, C12N 15/52, C12P 13/06, C12P 13/10, C12P 13/24

(54) **MICROORGANISM, AND METHOD FOR PRODUCING L-AMINO ACIDS USING SAME**

(30) Priority: 21.11.2024 KR 20240167777
(71) Applicant: CJ Cheiljedang Corporation, Seoul 04560 (KR)
(72) Inventor: KIM, Ju Eun, Seoul 04560 (KR); KIM, Ye-Eun, Seoul 04560 (KR); KIM, Kyungrim, Seoul 04560 (KR); LEE, Zeewon, Seoul 04560 (KR)
(74) Representative: Meissner Bolte Partnerschaft mbB
(86) International application number: PCT/KR2025/012159
(87) International publication number: WO 2026/111103

(57) **Abstract**

The present disclosure relates to a microorganism of the genus Corynebacterium in which the activity of fumarate hydratase is enhanced, and a method for producing L-amino acids using same. The microorganism of the genus Corynebacterium with enhanced fumarate hydratase exhibits increased production capability of L-amino acids such as L-arginine, L-citrulline, L-ornithine, O-acetylhomoserine, L-homoserine, and L-histidine, compared to a parent strain in which the fumarate hydratase is not enhanced, and the microorganism with enhanced fumarate hydratase can be widely used for the production of L-amino acids.

## Description

### [TECHNICAL FIELD]

### CROSS-REFERENCE TO RELATED APPLICATION(S)

The present disclosure claims the benefit of priority based on Korean Patent Application No. 10-2024-0167777, filed on November 21, 2024, and all contents disclosed in the documents of the corresponding Korean patent application are incorporated herein by reference.

The present disclosure relates to a microorganism having an enhanced fumarate hydratase activity and a method for producing an L-amino acid using the same.

### [BACKGROUND ART]

A microorganism of the genus *Corynebacterium* is a Gram-positive microorganism widely used for producing L-amino acids.

In order to produce L-amino acids and other useful substances, various studies for developing a microorganism having highly efficient productivity are being conducted. Specifically, for the production of L-arginine, a target substance-specific approach, such as increasing the expression of a gene encoding an enzyme mainly involved in L-arginine biosynthesis or removing unnecessary genes for the biosynthesis of L-arginine in a microorganism of the genus *Corynebacterium*, is mainly used (Korean Registered Patent No. 10-1102263).

However, there is still a need for research on a method that may efficiently produce L-amino acids with high efficiency.

### [DISCLOSURE]

### [TECHNICAL PROBLEM]

One embodiment of the present disclosure provides an L-amino acid-producing microorganism of the genus *Corynebacterium* with an enhanced activity of fumarate hydratase.

Another embodiment of the present disclosure provides a method for producing an L-amino acid, comprising culturing a microorganism of the genus *Corynebacterium* that produces L-amino acids and has an enhanced fumarate hydratase activity, and recovering the L-amino acid from the cultured microorganism, the medium, or both.

Another embodiment of the present disclosure provides a composition for producing an L-amino acid, comprising a microorganism of the genus *Corynebacterium* that produces L-amino acids and has enhanced fumarate hydratase activity.

Another embodiment of the present disclosure provides a use of an L-amino acid-producing microorganism of the genus *Corynebacterium* having an enhanced activity of fumarate hydratase, for producing an L-amino acid.

### [TECHNICAL SOLUTION]

This will be described in detail as follows. Meanwhile, each description and embodiment disclosed in the present disclosure may also be applied to other descriptions and embodiments. That is, all combinations of various elements disclosed in the present disclosure fall within the scope of this disclosure. In addition, the scope of the present disclosure should not be construed as being limited by the specific descriptions set forth below. Furthermore, those skilled in the art will recognize or ascertain, using no more than routine experimentation, many equivalents to the specific aspects of the present disclosure described herein. In addition, such equivalents are intended to be included in the present disclosure.

The present disclosure provides a microorganism with an enhanced L-amino acid production ability and a method for producing an L-amino acid using the same.

An embodiment of the present disclosure provides a microorganism producing an L-amino acid, having an enhanced activity of fumarate hydratase.

In the present disclosure, the term "fumarate hydratase" refers to an enzyme that catalyzes the reversible conversion of fumarate to L-malate. The fumarate hydratase of the present disclosure may be used interchangeably with fumarase and FumC protein. The fumarate hydratase may be derived from a microorganism of the genus *Corynebacterium*, specifically from *Corynebacterium glutamicum*.

In the present disclosure, the sequence of the fumarate hydratase may be available from GenBank of NCBI, which is a known database (e.g., NCBI Reference Sequence: WP_003856814.1). More specifically, the fumarate hydratase may have and/or comprise, consisting essentially of, or consist of the amino acid sequence of SEQ ID NO: 1.

In one embodiment, the fumarate hydratase may comprise not only the amino acid sequence of SEQ ID NO: 1 but also an amino acid sequence having a sequence identity or homology of at least 60%, 70%, 75%, 80%, 85%, 90%, 91%, 92%, 93%, 94%, 95%, 96%, 97%, 98%, 99%, 99.5%, or 99.9% or more with the amino acid sequence of SEQ ID NO: 1. In addition, as long as an amino acid sequence has such sequence identity or homology and has biological activity identical or corresponding to that of the fumarate hydratase of the present disclosure, an amino acid sequence in which a portion of the sequence is deleted, modified, substituted, conservatively substituted, or added may also be included within the scope of the present disclosure.

In one embodiment, the fumarate hydratase may be a polypeptide having fumarate hydratase activity encoded by a fumarate hydratase gene. The fumarate hydratase gene may be derived from a microorganism of the genus *Corynebacterium* (e.g., *Corynebacterium glutamicum*), but is not limited thereto. The sequence of the fumarate hydratase gene may be available from NCBI, which is a known database (e.g., NCBI Reference Sequence: NZ_CP016335.1, NCBI Reference Sequence: NC_003450.3).

In one embodiment, the fumarate hydratase gene encoding the fumarate hydratase may have, comprise, consist of, or consisting essentially of the nucleic acid sequence of SEQ ID NO: 2 or SEQ ID NO: 41.

In one embodiment, the fumarate hydratase gene may comprise or consist of a nucleic acid sequence having a homology or identity of at least 70%, 75%, 80%, 85%, 90%, 95%, 96%, 97%, 98%, 99%, 99.5%, 99.7%, or 99.9% or more with the nucleic acid sequence of SEQ ID NO: 2 or SEQ ID NO: 41.

In the present disclosure, the term "L-amino acid-producing microorganism" may be used to mean a case where a microorganism having an L-amino acid production ability has an increased L-amino acid production ability as the activity of fumarate hydratase is enhanced, and/or a case where a microorganism not having an L-amino acid production ability comes to have an L-amino acid production ability as the activity of fumarate hydratase is enhanced. In the present disclosure, "microorganism" encompasses unicellular bacteria and may be used interchangeably with "cell."

In the present disclosure, in order to distinguish the microorganism before the activity of fumarate hydratase is enhanced from the "L-amino acid-producing microorganism" in which, due to the enhancement of the activity of fumarate hydratase, the L-amino acid production ability is increased or the L-amino acid production ability is conferred, the microorganism before the activity of fumarate hydratase is enhanced may be expressed as a parent strain (parent microorganism or parent strain) or a host microorganism (host cell).

In one embodiment, the parent strain may be a wild-type, or one mutated to increase the L-amino acid production ability, for example, one in which the activity of a protein involved in the biosynthesis or metabolism of L-amino acids is regulated (increased/promoted or decreased/inhibited) as compared with the wild-type, but is not limited thereto. For example, the parent strain may be a wild-type microorganism of the genus *Corynebacterium* having an L-amino acid production ability, a microorganism of the genus *Corynebacterium* mutated to increase the production ability of glutamate-family amino acids, or a microorganism of the genus *Corynebacterium* mutated to increase the production ability of homoserine-family amino acids.

In one specific embodiment, as a result of enhancing the activity of fumarate hydratase in a known microorganism of the genus *Corynebacterium* mutated to increase an L-amino acid production ability, it was confirmed that an L-amino acid production ability may be further increased when the activity of fumarate hydratase is enhanced.

The L-amino acid may be any one of glutamate-family amino acids, homoserine-family amino acids, or L-histidine, and specifically, it may be any one or more selected from the group consisting of L-arginine, L-citrulline, L-ornithine, O-acetyl homoserine, L-homoserine, and L-histidine.

In the present disclosure, the term "glutamate-family amino acid" refers to an amino acid that may be biosynthesized using glutamate as a precursor. Specifically, the glutamate-family amino acid may be any one or more selected from the group consisting of L-ornithine, L-citrulline, L-arginine, and putrescine, but is not limited thereto as long as it is an amino acid that may be biosynthesized using glutamate as a precursor. The "glutamate-family amino acid" of the present disclosure may be used interchangeably with "glutamic acid-family amino acid."

In the present disclosure, the term "ornithine-family amino acids" refers to amino acids that may be biosynthesized using ornithine as a precursor. Specifically, the ornithine-family amino acids may be any one or more selected from the group consisting of L-ornithine, L-citrulline, and putrescine, but are not limited thereto as long as they are amino acids that may be biosynthesized using ornithine as a precursor.

In one embodiment, the host microorganism may be any microorganism of the genus *Corynebacterium* or of the genus *Escherichia* that is either a microorganism naturally having an L-amino acid production ability, or a parent strain into which a mutation has been introduced to acquire an L-amino acid production ability, the parent strain initially having no or remarkably low L-amino acid production ability. The microorganism of the genus *Corynebacterium* may include, but is not limited to, *Corynebacterium glutamicum*, *Corynebacterium stationis, Brevibacterium lactofermentum, Brevibacterium flavum, Corynebacterium thermoaminogenes, Corynebacterium efficiens*, and the like. More specifically, the microorganism of the genus *Corynebacterium* may be *Corynebacterium glutamicum*, and the strain of the genus *Escherichia* may be *Escherichia coli*.

In the present disclosure, the term "enhancement" of polypeptide activity means that the activity of the polypeptide is increased as compared to its endogenous activity. The enhancement may be used interchangeably with terms such as activation, up-regulation, overexpression, and increase. Here, activation, enhancement, up-regulation, overexpression, and increase may all include exhibiting an activity that was not originally possessed, or exhibiting an improved activity as compared to the endogenous activity or activity before modification. The "endogenous activity" refers to the activity of a specific polypeptide originally possessed by a parent strain or non-modified microorganism before trait change, when the trait is changed by genetic mutation due to natural or artificial factors. This may be used interchangeably with "activity before modification." The fact that the activity of a polypeptide is "enhanced," "up-regulated," "overexpressed," or "increased" as compared to the endogenous activity refers to an improvement as compared to the activity and/or concentration (expression level) of a specific polypeptide originally possessed by the parent strain or non-modified microorganism before trait change.

The enhancement may be achieved by introducing an exogenous polypeptide or through enhancement of activity and/or concentration (expression level) of an endogenous polypeptide. Whether the activity of the polypeptide is enhanced may be confirmed from an increase in the degree of activity of the corresponding polypeptide, in its expression level, or in the amount of the product produced by the corresponding polypeptide.

The enhancement of activity of the polypeptide can be achieved by applying various methods well known in the art, and it is not limited as long as the activity of the target polypeptide is enhanced beyond that of the microorganism before modification. Specifically, it may involve the use of genetic engineering and/or protein engineering, which are well known to those skilled in the art and are routine methods in molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase in the intracellular copy number of a polynucleotide encoding the polypeptide;
2) replacement of a gene expression regulatory region on a chromosome encoding the polypeptide with a sequence having strong activity;
3) modification of a nucleotide sequence encoding a start codon or 5'-UTR region of a gene transcript encoding the polypeptide;
4) modification of the amino acid sequence of the polypeptide to enhance the polypeptide activity;
5) modification of the polynucleotide sequence encoding the polypeptide to enhance the polypeptide activity (for example, modification of the polynucleotide sequence of the polypeptide gene so as to encode a polypeptide modified to enhance the activity of the polypeptide);
6) introduction of an exogenous polypeptide or an exogenous polynucleotide encoding the same exhibiting the activity of the polypeptide;
7) codon optimization of a polynucleotide encoding the polypeptide;
8) analysis of the tertiary structure of a polypeptide to select an exposed site and modify or chemically modify the same;
9) regulation of intracellular localization of the polypeptide; or
10) a combination of two or more selected from the group consisting of the items 1) to 9) above, but is not particularly limited thereto.

More specifically,
The increase in the intracellular copy number of a polynucleotide encoding the polypeptide as described in item 1) may be achieved by introduction into a host cell of a vector in which the polynucleotide encoding the corresponding polypeptide is operably linked and which may replicate and function independently of the host. Alternatively, it may be achieved by introduction of one copy or two or more copies of the polynucleotide encoding the corresponding polypeptide into the chromosome of the host cell. The introduction into the chromosome may be performed by introducing into the host cell a vector capable of inserting the polynucleotide into the chromosome of the host cell, but is not limited thereto. The vector is as described above.

The replacement of a gene expression regulatory region (or expression regulatory sequence) on a chromosome encoding the polypeptide with a sequence having strong activity as described in item 2) may be, for example, generating a mutation in the sequence by deletion, insertion, non-conservative or conservative substitution, or a combination thereof, so as to further enhance the activity of the expression regulatory region, or replacement with a sequence having stronger activity. The expression regulatory region may include, but is not particularly limited to, a promoter, an operator sequence, a sequence encoding a ribosome binding site, and a sequence regulating the termination of transcription and translation. In one embodiment, it may be replacing the original promoter with a strong promoter, but is not limited thereto.

Examples of known strong promoters include CJ1 to CJ7 promoters (US Patent No. 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. 10584338 B2), O2 promoter (US Patent No. 10273491 B2), tkt promoter, yccA promoter, etc., but are not limited thereto.

The modification of a nucleotide sequence encoding a start codon or 5'-UTR region of a gene transcript encoding the polypeptide as described in item 3) may, for example, involve substituting the nucleotide sequence with a nucleotide sequence encoding another start codon having a polypeptide expression rate higher than that of the endogenous start codon, but is not limited thereto.

The modification of the amino acid sequence or polynucleotide sequence as described in items 4) and 5) may be, but is not limited to, the generation of a mutation in the amino acid sequence of the polypeptide or the polynucleotide sequence encoding the polypeptide by deletion, insertion, non-conservative or conservative substitution, or a combination thereof, so as to enhance the activity of the polypeptide, or replacement with an amino acid sequence or polynucleotide sequence improved to have stronger activity or an amino acid sequence or polynucleotide sequence improved to have increased activity. The replacement may be specifically performed by inserting a polynucleotide into a chromosome by homologous recombination, but is not limited thereto. In this case, the vector used may further include a selection marker for confirming whether the polynucleotide has been inserted into the chromosome. The selection marker is as described above.

The introduction of an exogenous polynucleotide exhibiting the activity of the polypeptide as described in item 6) may be the introduction of an exogenous polynucleotide encoding a polypeptide exhibiting the same/similar activity as the polypeptide into a host cell. There is no limitation on the origin or sequence thereof as long as it exhibits the same/similar activity as the polypeptide. The method used for the introduction may be appropriately selected from known transformation methods and performed by those skilled in the art, and the polypeptide is produced by expressing the introduced polynucleotide in the host cell, whereby its activity may be increased.

The codon optimization of a polynucleotide encoding the polypeptide as described in item 7) may be a codon optimization of an endogenous polynucleotide such that transcription or translation in the host cell is increased, or an optimization of codons of an exogenous polynucleotide such that optimized transcription and translation are performed in the host cell.

The analysis of the tertiary structure of a polypeptide to select an exposed site and modify or chemically modify it as described in item 8) may be, for example, determining a template protein candidate according to the degree of sequence similarity by comparing the sequence information of the polypeptide to be analyzed with a database in which sequence information of known proteins is stored, and confirming the structure based on the same, and then selecting an exposed site to be modified or chemically modified, and performing the modification or chemical modification.

The regulation of intracellular localization of the polypeptide as described in item 9) may involve targeting the polypeptide to a specific organelle or a specific intracellular space. For example, it may involve targeting the polypeptide to the periplasm or cytoplasm through addition or removal of a leader sequence functioning in the targeting of the polypeptide, but is not limited thereto.

Such enhancement of polypeptide activity may be that the activity level or the concentration (expression level) of the corresponding polypeptide is increased based on the activity or concentration of the polypeptide expressed in the wild-type or the microorganism strain before modification, or that the amount of the product produced from the corresponding polypeptide is increased, but is not limited thereto.

In one specific embodiment of the present invention, the activity of the fumarate hydratase may be enhanced as compared with a non-mutated microorganism by increasing the copy number or enhancing the activity of a promoter.

Enhancing the activity of the promoter may be enhancing the activity thereof by introducing a promoter exhibiting improved activity for fumarate hydratase (FumC). As a specific example of the present invention, the promoter exhibiting improved activity includes, without limitation, a promoter having increased activity as compared with the *fumC* native promoter. Such promoters include, without limitation, a promoter of a gene having activity higher than the gene expression-inducing activity of the *fumC* native promoter, or a mutant promoter having increased activity through gene mutation of the *fumC* native promoter, or the like. Specifically, the promoter exhibiting improved activity of the present invention may be the SPL13 promoter. Specifically, the SPL13 promoter may be composed of the nucleotide sequence of SEQ ID NO: 9, however, it may also be a nucleotide sequence having a homology of 70% or more, specifically 80% or more, and more specifically 90% or more with the nucleotide sequence of SEQ ID NO: 9.

The increase in the copy number of the gene is not particularly limited thereto, but may be performed in a form operably linked to a vector or by being inserted into a chromosome in a host cell. Specifically, a vector in which a polynucleotide encoding fumarate hydratase, which is the target protein for enhancement in the present disclosure, is operably linked and which may replicate and function independently of the host, may be introduced into a host cell. Alternatively, a vector in which the polynucleotide is operably linked and which is capable of inserting the polynucleotide into the chromosome in the host cell, may be introduced into the chromosome of a host cell. The insertion of the polynucleotide into the chromosome may be performed by any method known in the art, for example, by homologous recombination. Since the vector of the present disclosure may be inserted into a chromosome by inducing homologous recombination, it may further include a selection marker for confirming whether the polynucleotide has been inserted into the chromosome. The selection marker is for selecting cells transformed with the vector to confirm whether a target polynucleotide is inserted, and markers that confer selectable phenotypes such as drug resistance, auxotrophy, resistance to cytotoxic agents, or expression of surface proteins may be used, but are not limited thereto. In an environment in the presence of a selective agent, only cells expressing the selection marker survive or exhibit other phenotypes, so that transformed cells may be selected.

In the present disclosure, the expression that a polynucleotide (which may be used interchangeably with "gene") or polypeptide (which may be used interchangeably with "protein") "comprises a specific nucleic acid sequence or amino acid sequence, consists of a specific nucleic acid sequence or amino acid sequence, or is represented by a specific nucleic acid sequence or amino acid sequence" may mean that the polynucleotide or polypeptide essentially includes the specific nucleic acid sequence or amino acid sequence, and it may also be interpreted as including a "substantially equivalent sequence" in which a mutation (deletion, substitution, modification, and/or addition) is introduced into the specific nucleic acid sequence or amino acid sequence within the scope of maintaining the original function and/or desired function of the polynucleotide or polypeptide (or as not excluding such mutations).

In one embodiment, the nucleic acid sequence or amino acid sequence provided in the present disclosure may include sequences modified by conventional mutagenesis methods, for example, directed evolution and/or site-directed mutagenesis, etc., within a range in which their original function or desired function is maintained. In one embodiment, the statement that a polynucleotide or polypeptide "comprises a specific nucleic acid sequence or amino acid sequence or consists of a specific nucleic acid sequence or amino acid sequence" may mean that the polynucleotide or polypeptide (i) essentially comprises the specific nucleic acid sequence or amino acid sequence, or (ii) consists of or essentially comprises a nucleic acid sequence or an amino acid sequence having a homology of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the specific nucleic acid sequence or amino acid sequence, and maintains the original function and/or desired function. In the present disclosure, the desired function may mean a function of increasing or conferring the L-amino acid production ability of a microorganism.

The nucleic acid sequence described in the present disclosure may be subjected to various modifications in the coding region within a range that does not change the amino acid sequence and/or function of the protein expressed from the coding region, in consideration of codons preferred in a microorganism in which the protein (lysine efflux protein) is to be expressed due to codon degeneracy.

In the present disclosure, the term "homology (identity)" refers to the degree of consistency with a given nucleic acid sequence or amino acid sequence and may be expressed as a percentage (%). In the case of homology to a nucleic acid sequence, it may be determined, for example, using the algorithm BLAST (e.g., as described in literature, see: Karlin and Altschul, Pro. Natl. Acad. Sci. USA, 90, 5873, 1993) or FASTA by Pearson (see: Methods Enzymol., 183, 63, 1990). Based on the algorithm BLAST, programs called BLASTN or BLASTX have been developed (see: http://www.ncbi.nlm.nih.gov).

In one embodiment, a polynucleotide comprising a specific nucleic acid sequence provided in the present disclosure may be interpreted as comprising not only the specific nucleic acid sequence or a nucleic acid sequence substantially equivalent thereto, but also a polynucleotide fragment comprising a nucleic acid sequence complementary to the specific nucleic acid sequence. Specifically, the polynucleotide having the complementarity may be subjected to hybridization at a Tm value appropriately adjustable by those skilled in the art according to the purpose, for example, a Tm value of 55°C, 60°C, 63°C, or 65°C, and subsequently analyzed under the conditions described below: such conditions are specifically described in known literature. For example, conditions in which genes having high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more hybridize with each other, and genes having complementarity lower than that do not hybridize, or washing conditions for conventional Southern hybridization such as 60°C, 1x SSC (saline-sodium citrate buffer), and 0.1% (w/v) SDS (Sodium Dodecyl Sulfate); under salt concentration and temperature conditions corresponding to 60°C, 0.1x SSC, and 0.1% SDS; or under salt concentration and temperature conditions corresponding to 68°C, 0.1x SSC, and 0.1% SDS, with washing performed once, specifically two to three times, may be enumerated, but is not limited thereto. Hybridization requires that two nucleotides have complementary sequences, or a mismatch between bases may be allowed according to the stringency of hybridization. The term "complementary" may be used to describe a relationship between nucleotide bases capable of hybridizing with each other. For example, in the case of DNA, adenosine is complementary to thymine and cytosine is complementary to guanine. The appropriate stringency for hybridizing polynucleotides depends on the length and degree of complementarity of the polynucleotides, which is well known in the relevant technical field (see Sambrook et al., supra, 9.50-9.51, 11.7-11.8).

The introduction of the polynucleotide or vector may be performed by appropriately selecting a known transformation method by those skilled in the art. In the present disclosure, the term "transformation" means introducing a vector comprising a polynucleotide encoding a target protein (exogenous protein) into a host cell so that the protein encoded by the polynucleotide may be expressed in the host cell. The transformed polynucleotide may include all such cases regardless of whether they are inserted and located within the chromosome of the host cell or located outside the chromosome, as long as they may be expressed in the host cell. In addition, the polynucleotide includes DNA and/or RNA encoding the target protein. The form in which the polynucleotide is introduced is not limited as long as it may be introduced and expressed into the host cell. For example, the polynucleotide may be introduced into the host cell in the form of an expression cassette, which is a gene construct comprising all elements necessary for expression by itself. The expression cassette may usually include expression regulatory elements such as a promoter, a transcription termination signal, a ribosome binding site, and/or a translation termination signal operably linked to the polynucleotide. The expression cassette may be in the form of a self-replicable expression vector. In addition, the polynucleotide may be introduced into the host cell in its own form and operably linked to sequences necessary for expression in the host cell.

In the above, the term "operably linked" may mean that an expression regulatory element and a polynucleotide are functionally connected so that the expression regulatory element may perform transcriptional control (e.g., transcription initiation) of the polynucleotide encoding the target protein of the present disclosure. Operable linkage may be performed using recombinant DNA techniques known in the art, and for example, may be performed by conventional site-specific DNA cleavage and linkage, but is not limited thereto.

In the present disclosure, modifying an expression regulatory sequence so that the expression of the polynucleotide is increased is not particularly limited thereto, but may be performed by inducing mutation in the sequence by deletion, insertion, non-conservative or conservative substitution, or a combination thereof, so as to further enhance the activity of the expression regulatory sequence, or by replacement with a nucleic acid sequence having stronger activity. Specifically, it may be performed by replacing with a strong promoter. The expression regulatory sequence may include, but is not particularly limited to, a promoter, an operator sequence, a sequence encoding a ribosome binding site, and a sequence regulating the termination of transcription and translation, and the like.

A strong promoter may be connected upstream of the polynucleotide expression unit instead of the original promoter, and it is not limited thereto. Examples of known strong promoters may include CJ1 to CJ7 promoters (US Patent No. 7662943 B2), SPL1, SPL7 or SPL13 promoters (US Patent No. 10584338 B2), O2 promoter (US Patent No. 10273491 B2), PgapA promoter, lac promoter, trp promoter, trc promoter, tac promoter, lambda phage PR promoter, PL promoter, and tet promoter.

The method of transforming the polynucleotide into a host cell may be performed by any method of introducing a nucleic acid into a cell (microorganism), and may be performed by appropriately selecting a transformation technique known in the art by those skilled in the art. Examples of the known transformation methods include electroporation, calcium phosphate (CaPO₄) precipitation method, calcium chloride (CaCl₂) precipitation method, microinjection, polyethylene glycol (PEG) precipitation method (polyethylene glycol-mediated uptake), DEAE-dextran method, cationic liposome method, lipofection, lithium acetate-DMSO method, etc., but are not limited thereto.

The introduction (insertion) of the polynucleotide into the host cell genome (chromosome) may be performed by those skilled in the art by appropriately selecting a known method, for example, using an RNA-guided endonuclease system (RNA-guided endonuclease system or CRISPR system; for example, one or more selected from the group consisting of a mixture, a complex (e.g., a ribonucleoprotein (RNP)), and a recombinant vector (e.g., a vector comprising both an RNA-guided endonuclease encoding gene and a guide RNA encoding DNA, etc.), wherein the mixture comprises (a) an RNA-guided endonuclease (e.g., Cas9 protein, etc.), an encoding gene thereof, or a vector comprising the gene; and (b) a guide RNA (e.g., single guide RNA (sgRNA), etc.), an encoding DNA thereof, or a vector comprising the DNA (e.g., a mixture of an RNA-guided endonuclease protein and a guide RNA, etc.), but is not limited thereto.

In the present disclosure, the term "vector" refers to a DNA construct containing a nucleotide sequence of a polynucleotide encoding a target protein operably linked to a suitable regulatory sequence so that the target protein may be expressed in a suitable host. The regulatory sequence may include a promoter capable of initiating transcription, any operator sequence for controlling transcription, a sequence encoding a suitable mRNA ribosome binding site, and/or a sequence controlling termination of transcription and/or translation. After being transformed into a suitable host cell, the vector may be expressed independently of the host cell's genome or integrated into the host cell's genome.

The vector usable in the present disclosure is not particularly limited as long as it can replicate in a host cell, and may be selected from any commonly used vectors. Examples of commonly used vectors include plasmids, cosmids, viruses, bacteriophages, and the like, in a natural or recombinant state. For example, as a phage vector or cosmid vector, pWE15, M13, MBL3, MBL4, IXII, ASHII, APII, t10, t11, Charon4A, and Charon21A, and the like, may be used, and as a plasmid vector, pBR-based, pUC-based, pBluescriptII-based, pGEM-based, pTZ-based, pCL-based, and pET-based, and the like, may be used. Specifically, vectors such as pDZ, pDC, pDCM2, pDC24, pACYC177, pACYC184, pCL, pECCG117, pUC19, pBR322, pMW118, pCC1BAC, and pCES208 may be exemplified, but are not limited thereto.

In addition, one embodiment of the present disclosure provides a microorganism that produces L-amino acids in which the activity of fumarate hydratase is enhanced, and additionally, the activities of malate:quinone oxidoreductase, malate dehydrogenase, aspartate transaminase, and NADP-specific glutamate dehydrogenase are enhanced as compared with a non-modified microorganism.

In the present disclosure, the term "malate:quinone oxidoreductase" is an enzyme in the TCA cycle and refers to an enzyme that catalyzes the reversible conversion of malate and quinone to quinol and oxaloacetate. The malate:quinone oxidoreductase of the present disclosure may be used interchangeably with MQO protein, and in the present disclosure, the sequence of the malate:quinone oxidoreductase may be obtained from GenBank of NCBI, which is a known database. More specifically, the malate:quinone oxidoreductase may have and/or comprise, or essentially consist of, or be composed of the amino acid sequence of SEQ ID NO: 32.

For example, a protein consisting of the amino acid sequence of SEQ ID NO: 32 may refer to a protein endogenously present in a microorganism of the genus *Corynebacterium* encoded by the known NCgl1926 gene, but is not limited thereto. Specifically, the protein consisting of the amino acid sequence of SEQ ID NO: 32 may refer to the malate:quinone oxidoreductase derived from *Corynebacterium glutamicum* ATCC13032 encoded by the NCgl1926 gene, but is not limited thereto.

In addition, the malate:quinone oxidoreductase of the present disclosure may comprise the amino acid sequence of SEQ ID NO: 32, as well as an amino acid sequence having a sequence identity or homology of 60% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the sequence of SEQ ID NO: 32. In addition, as long as an amino acid sequence has such sequence identity or homology and has biological activity identical or corresponding to that of the malate:quinone oxidoreductase of the present disclosure, an amino acid sequence in which a portion of the sequence is deleted, modified, substituted, conservatively substituted, or added may also be included within the scope of the present disclosure.

In the present disclosure, the term "malate dehydrogenase" is an enzyme in the TCA cycle and refers to an enzyme that reversibly catalyzes the oxidation of malate to oxaloacetate by reducing NAD+ to NADH. The malate dehydrogenase of the present disclosure may be used interchangeably with MDH protein, and in the present disclosure, the sequence of the malate dehydrogenase may be obtained from GenBank of NCBI, which is a known database. More specifically, the malate dehydrogenase may have and/or comprise, or essentially consist of, or be composed of the amino acid sequence of SEQ ID NO: 34.

For example, a protein consisting of the amino acid sequence of SEQ ID NO: 34 may refer to an endogenous protein in a microorganism of the genus *Corynebacterium* encoded by the known NCgl0631 gene, but is not limited thereto. Specifically, the protein consisting of the amino acid sequence of SEQ ID NO: 34 may refer to malate dehydrogenase derived from *Corynebacterium glutamicum* ATCC13032 encoded by the NCgl0631 gene, but is not limited thereto.

In addition, the malate dehydrogenase of the present disclosure may comprise the amino acid sequence of SEQ ID NO: 34, as well as an amino acid sequence having a sequence identity or homology of 60% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the sequence of SEQ ID NO: 34. In addition, as long as an amino acid sequence has such sequence identity or homology and has biological activity identical or corresponding to that of the malate dehydrogenase of the present disclosure, an amino acid sequence in which a portion of the sequence is deleted, modified, substituted, conservatively substituted, or added may also be included within the scope of the present disclosure.

In the present disclosure, the term "aspartate transaminase" refers to an enzyme that catalyzes a reaction for producing L-glutamic acid and oxaloacetate by transferring the amino group of L-aspartic acid to 2-oxoglutaric acid. The aspartate transaminase of the present disclosure may be used interchangeably with AspB protein, and in the present disclosure, the sequence of the aspartate transaminase may be obtained from GenBank of NCBI, which is a known database. More specifically, the aspartate transaminase may have and/or comprise, essentially consist of, or be composed of the amino acid sequence of SEQ ID NO: 36.

For example, a protein consisting of the amino acid sequence of SEQ ID NO: 36 may refer to an endogenous protein in a microorganism of the genus *Corynebacterium* encoded by the known NCgl0237 gene, but is not limited thereto. Specifically, the protein consisting of the amino acid sequence of SEQ ID NO: 36 may refer to the aspartate transaminase derived from *Corynebacterium glutamicum* ATCC13032 encoded by the NCgl0237 gene, but is not limited thereto.

In addition, the aspartate transaminase of the present disclosure may comprise the amino acid sequence of SEQ ID NO: 36 as well as an amino acid sequence having a sequence identity or homology of 60% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the sequence of SEQ ID NO: 36. In addition, as long as an amino acid sequence has such sequence identity or homology and has biological activity identical or corresponding to that of the aspartate transaminase of the present disclosure, an amino acid sequence in which a portion of the sequence is deleted, modified, substituted, conservatively substituted, or added may also be included within the scope of the present disclosure.

In the present disclosure, the term "NADP-specific glutamate dehydrogenase" refers to an enzyme that catalyzes a reaction for converting glutamate to 2-oxoglutarate and ammonia in an NADP-dependent manner. The NADP-specific glutamate dehydrogenase of the present disclosure may be used interchangeably with GDH protein, and in the present disclosure, the sequence of the NADP-specific glutamate dehydrogenase may be obtained from GenBank of NCBI, which is a known database. More specifically, the NADP-specific glutamate dehydrogenase may have and/or comprise, essentially consist of, or be composed of the amino acid sequence of SEQ ID NO: 38.

For example, the protein consisting of the amino acid sequence of SEQ ID NO: 38 may refer to an endogenous protein in a microorganism of the genus *Corynebacterium* encoded by the known NCgl1999 gene, but is not limited thereto. Specifically, the protein consisting of the amino acid sequence of SEQ ID NO: 38 may refer to NADP-specific glutamate dehydrogenase derived from *Corynebacterium glutamicum* ATCC13032 encoded by the NCgl1999 gene, but is not limited thereto.

In addition, the NADP-specific glutamate dehydrogenase of the present disclosure may comprise the amino acid sequence of SEQ ID NO: 38 as well as an amino acid sequence having 60% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more sequence identity or homology with the sequence of SEQ ID NO: 38. In addition, as long as an amino acid sequence has such sequence identity or homology and has biological activity identical or corresponding to that of the NADP-specific glutamate dehydrogenase of the present disclosure, an amino acid sequence in which a portion of the sequence is deleted, modified, substituted, conservatively substituted, or added may also be included within the scope of the present disclosure.

In one specific embodiment, in order to increase L-amino acid production ability, it was confirmed that the L-amino acid production ability increases as a result of simultaneously enhancing the activity of malate:quinone oxidoreductase, malate dehydrogenase, aspartate transaminase, and NADP-specific glutamate dehydrogenase, in addition to the enhancement of fumarate hydratase activity. Specifically, it was confirmed that the L-amino acid production ability of an L-amino acid-producing microorganism into which a gene combination encoding fumarate hydratase, malate:quinone oxidoreductase, malate dehydrogenase, aspartate transaminase, and NADP-specific glutamate dehydrogenase was introduced using a plasmid was further improved.

The above-described "L-amino acid," "microorganism," "enhancement of activity," etc. are as described above.

Another embodiment provides a method for increasing L-amino acid production ability of the microorganism, or a method for conferring L-amino acid production ability to the microorganism, comprising enhancing the activity of fumarate hydratase of the microorganism.

The step of enhancing the activity of fumarate hydratase of the microorganism may comprise introducing a mutation so as to express a polypeptide having the activity of fumarate hydratase in the microorganism or connecting a strong promoter upstream of a polynucleotide expression unit encoding the polypeptide.

The step of introducing the mutation may comprise introducing (transforming) a polynucleotide encoding a polypeptide having the activity of fumarate hydratase or a recombinant vector comprising the polynucleotide into the microorganism.

Another embodiment provides a method for producing an L-amino acid, comprising culturing the above-described microorganism having enhanced fumarate hydratase activity in a medium. The method may further comprise, after the culturing, recovering the L-amino acid from the cultured microorganism, the medium, or both.

In the method, the culturing of the microorganism is not particularly limited thereto, but may be performed by a known batch culture method, continuous culture method, fed-batch culture method, etc. At this time, the culture conditions are not particularly limited thereto, but a target pH (e.g., pH 5 to 9, specifically pH 6 to 8, most specifically pH 6.8) may be adjusted using a basic compound (e.g., sodium hydroxide, potassium hydroxide, or ammonia) or an acidic compound (e.g., phosphoric acid or sulfuric acid), and aerobic conditions may be maintained by introducing oxygen or an oxygen-containing gas mixture into the culture. The culture temperature may be maintained at 20 to 45°C, or 25 to 40°C, and culturing may be performed for about 10 to 160 hours, but is not limited thereto. By the culture, the L-amino acid may be secreted into the medium or remain in the cells.

The medium usable for the culture may comprise, as a carbon source, one or more selected from the group consisting of sugars and carbohydrates (e.g., glucose, sucrose, lactose, fructose, maltose, molasses, starch, and cellulose), oils and fats (e.g., soybean oil, sunflower oil, peanut oil, and coconut oil), fatty acids (e.g., palmitic acid, stearic acid, and linoleic acid), alcohols (e.g., glycerol and ethanol), organic acids (e.g., acetic acid), etc., which may be used individually, or in a mixture of two or more types thereof, but is not limited thereto. As a nitrogen source, one or more selected from the group consisting of nitrogen-containing organic compounds (e.g., peptone, yeast extract, meat extract, malt extract, corn steep liquor, soybean meal, and urea), inorganic compounds (e.g., ammonium sulfate, ammonium chloride, ammonium phosphate, ammonium carbonate, and ammonium nitrate), etc. may be used individually, or in a mixture of two or more types thereof, but is not limited thereto. As a phosphorus source, one or more selected from the group consisting of monopotassium phosphate, dipotassium hydrogen phosphate, and corresponding sodium-containing salts, etc. may be used individually, or in a mixture of two or more types thereof, but is not limited thereto. In addition, the medium may comprise essential growth-promoting substances such as other metal salts (e.g., magnesium sulfate or iron sulfate), amino acids, and/or vitamins.

The recovering of the L-amino acid may be collecting the target amino acid from the medium, culture broth, or microorganism using a suitable method known in the art according to the culture method. For example, the recovery may be performed by one or more methods selected from the group consisting of centrifugation, filtration, anion exchange chromatography, crystallization, and HPLC, and the like. The method for recovering the L-amino acid may additionally comprise a purification step before, simultaneously with, or after the recovery.

The "L-amino acid," "microorganism," "enhancement of activity," etc. are as described above.

Another embodiment of the present disclosure provides a composition for producing an L-amino acid comprising a microorganism of the genus *Corynebacterium* that produces an L-amino acid and has enhanced fumarate hydratase activity.

The composition of the present disclosure may further comprise any suitable excipient commonly used in a composition for producing an amino acid, and such excipient may be, for example, a preservative, a wetting agent, a dispersing agent, a suspending agent, a buffering agent, a stabilizing agent, or an isotonic agent, but is not limited thereto.

In the composition of the present disclosure, the "L-amino acid," "microorganism," "enhancement of activity," etc. are as described above.

Still another embodiment of the present disclosure provides a use of a microorganism of the genus *Corynebacterium* according to the present disclosure, having enhanced fumarate hydratase activity and producing an L-amino acid, for producing an L-amino acid.

The "L-amino acid," "microorganism," "enhancement of activity," etc. are as described above.

Still another aspect of the present disclosure provides a use of an L-amino acid-producing microorganism of the genus *Corynebacterium* according to the present disclosure having enhanced fumarate hydratase activity for preparing a composition for producing an L-amino acid.

The "L-amino acid," "microorganism," "enhancement of activity," and the like are as described above.

According to another aspect of the present disclosure, the present disclosure provides a composition, method, product, process, or use characterized by one or more elements disclosed in the present disclosure.

### [ADVANTAGEOUS EFFECTS]

The present disclosure provides a technology for increasing the L-amino acid production ability of a microorganism, and for this purpose, provides a fumarate hydratase-enhanced microorganism, and it was confirmed that the fumarate hydratase-enhanced microorganism has increased L-amino acid production ability as compared with a parent strain in which fumarate hydratase is not enhanced, so that the fumarate hydratase-enhanced microorganism may be widely utilized for L-amino acid production.

### [MODE FOR INVENTION]

Hereinafter, the present disclosure will be described in more detail by way of examples. However, the following examples are only preferred embodiments for illustrating the present disclosure, and therefore, it is not intended to be construed as limiting the scope of rights of the present disclosure thereto. Meanwhile, technical matters not described in the present disclosure may be sufficiently understood and easily implemented by a person of ordinary skill in the art to which the present disclosure pertains or a similar technical field.

### Example 1. Construction of a plasmid for fumarate hydratase enhancement

In order to confirm the effect of enhancement of the fumarate hydratase (fumarate hydratase; hereinafter fumC) gene of *Corynebacterium glutamicum* on L-amino acid production, an expression vector for the BBD29_RS05465 gene (SEQ ID NO: 2), which is NCBI Accession No. NZ_CP016335.1 derived from *Corynebacterium glutamicum* ATCC13869, was prepared as follows.

Specifically, in order to insert the fumarate hydratase gene into the *Corynebacterium glutamicum* chromosome, BBD29_RS10775 (SEQ ID NO: 4), known as a gene encoding a transposon in *Corynebacterium glutamicum*, was used as an insertion site (Journal of Biotechnology 104, 5-25 Jorn Kalinowski et al, 2003). In order to replace the BBD29_RS10775 gene with a fumC-enhanced form, a BBD29_RS10775 deletion and target gene insertion vector was prepared. To prepare the vector, PCR was performed using the chromosome of ATCC13869 as a template and each of the primer pairs of SEQ ID NO: 5 and SEQ ID NO: 6, and the primer pair of SEQ ID NO: 7 and SEQ ID NO: 8.

PfuUltraTM high-fidelity DNA polymerase (Stratagene) was used as a polymerase for the PCR reaction. As PCR conditions, denaturation at 95°C for 30 seconds; annealing at 55°C for 30 seconds; and extension at 72°C for 1 minute were performed, and denaturation, annealing, and extension under these conditions were repeated 28 times. As a result, DNA fragments of 756 bp and 731 bp were obtained, respectively. The obtained DNA products were purified using a PCR purification kit (PCR Purification kit, QUIAGEN), and the purified amplification product was treated with restriction enzyme SmaI, and then cloned using a pDC24 vector (SEQ ID NO: 146) heat-treated at 65°C for 20 minutes and an In-Fusion cloning kit (Infusion Cloning Kit, TaKaRa) according to the provided manual, thereby preparing a BBD29_RS10775 deletion and target gene insertion vector, pDC24ΔBBD29_RS10775.

In order to enhance the activity of fumarate hydratase (fumarate hydratase_FumC), a plasmid for enhancing the fumC gene was prepared using the Pspl13 promoter (SEQ ID NO: 9, Korean Registered Patent No. 10-1783170), which is a promoter known as a strong promoter.

Specifically, to prepare a gene construct in which the fumC gene is linked to the Pspl13 promoter, PCR was performed on the gene fragment of the downstream region of the fumC gene using the chromosome of *Corynebacterium glutamicum* ATCC13869 as a template and primers of SEQ ID NO: 10 and SEQ ID NO: 11, respectively, under the same conditions as the PCR conditions used in the method for preparing the pDC24ΔBBD29_RS10775 vector.

PfuUltra^{TM} high-fidelity DNA polymerase (Stratagene) was used as a polymerase for the PCR reaction, and as PCR conditions, 30 seconds of denaturation at 95°C; 30 seconds of annealing at 55°C; and 1 minute of polymerization at 72°C were performed, and denaturation, annealing, and polymerization under these conditions were repeated 28 times. As a result, a 1450 bp DNA fragment of *Corynebacterium glutamicum* ATC13869 fumC was obtained. PCR was performed with the primers of SEQ ID NO: 10 and SEQ ID NO: 13 using the Pspl13 promoter (SEQ ID NO: 9, Korean Registered Patent No. 10-1783170) and the amplified fumC gene DNA fragment as a template. As PCR conditions, after denaturation at 95°C for 5 minutes, 28 cycles of denaturation at 95°C for 30 seconds; annealing at 55°C for 30 seconds; and polymerization at 72°C for 2 minutes were repeated, and then extension was performed at 72°C for 5 minutes.

As a result, a 1738 bp Pspl13_fumC DNA fragment, comprising the Pspl13 promoter and encoding fumC, was amplified. The amplification product was purified using a PCR purification kit (PCR Purification kit, QIAGEN) and used as an insert DNA fragment for vector preparation. After treating the purified amplification product with restriction enzyme SmaI, the molar concentration (M) ratio of the pDC24ΔBBD29_RS10775 vector heat-treated at 65°C for 20 minutes to the insert DNA fragment, which is the amplification product, was adjusted to 1:2, and cloning was performed using the In-Fusion^{®} HD cloning kit (Clontech) according to the provided manual, thereby preparing a vector pDC24ΔBBD29_RS10775::Pspl13-fumC for introducing Pspl13-fumC into the chromosome.

The sequences of the primers used in said Example 1 are shown in Table 1 below.

**[TABLE 1]**

| Name | Sequence (5'-> 3') | SEQ ID NO |
|---|---|---|
| RS10775-up-F | AAACGACGGCCAGTGAATTCTGGCCATAAC GAGTGCGGTA | SEQ ID NO: 5 |
| RS10775-up-R | GGCTCTTCCTGTTTAGTACTAAACCGGAAccc GGGCCTTCACCCC | SEQ ID NO: 6 |
| RS10775-down-F | GACGGATTCAGGGGGTGAAGGCCCgggTTCC GGTTTAGTACTA | SEQ ID NO: 7 |
| RS10775-down-R | CTTGCATGCCTGCAGGTCGACTGCTGGAGT GAGCGCATGA | SEQ ID NO: 8 |
| *fumC*-3'-F | GATTATTGGAGGAGATCAAAACAATGACCG AGCAGGAATTCC | SEQ ID NO: 10 |
| *fumC*-3'-R | GGCTCTTCCTGTTTAGTTTAGAACTTGTTCT CGCGCT | SEQ ID NO: 11 |
| Pspl13-F | GGCCCTTCCGGTTTAGTGGCGCTTCATGTCA ACAATCTTTAAC | SEQ ID NO: 12 |
| Pspl13-R | GGAATTCCTGCTCGGTCATTGTTTTGATCTC CTCCAATAATC | SEQ ID NO: 13 |

### Comparative Example 1. Preparation of microorganisms producing glutamate-family amino acids and in which fumarate hydratase is non-enhanced

As a control for confirming the effect of increasing productivity of glutamate-family amino acids according to fumarate hydratase enhancement, *Corynebacterium glutamicum* CJR2 strain, which is a *Corynebacterium glutamicum* ATCC13869 strain wherein the argR gene was deleted and an argB(M54V) gene mutation was introduced, and *Corynebacterium glutamicum* CJR100 strain, which is a *Corynebacterium glutamicum* ATCC13869 strain wherein the argR gene was deleted and an argB(M54V) gene mutation and argC gene enhancement were introduced, were prepared as follows (Ikeda, Masato et al., Applied and environmental microbiology 75(6)1635-41, 2009).

### Comparative Example 1-1. Preparation of a microorganism in which the argR gene is deleted and the argB(M54V) mutation is introduced

A vector for introducing the argR deletion and argB(M54V) mutation was prepared as follows. PCR using primer pairs of SEQ ID NOs: 14 and 15, and SEQ ID NOs: 16 and 17, and overlapping PCR using the primer pair of SEQ ID NOs: 14 and 17 were performed using the genomic DNA of *Corynebacterium glutamicum* ATCC13869 as a template to obtain a homologous recombination fragment having the argR deletion mutation sequence. In order to prepare a homologous recombination fragment having the argB(M54V) mutation in the same method as above, PCR using primer pairs of SEQ ID NOs: 18 and 19, and SEQ ID NOs: 20 and 21, and overlapping PCR using the primer pair of SEQ ID NOs: 18 and 21 were performed. The PCR reaction was performed by repeating 30 cycles of denaturation at 95°C for 30 seconds; annealing at 55°C for 30 seconds; and extension at 72°C for 2 minutes. Then, the linearized pDC24 vector and each homologous recombination fragment were fusion cloned in the same method as described in Example 1. The prepared vectors (recombinant plasmids) were named pDC24-ΔargR and pDC24-argB(M54V), respectively.

In order to introduce the argR deletion mutation into wild-type *Corynebacterium glutamicum* ATCC13869, the prepared pDC24-ΔargR plasmid was transformed into the *Corynebacterium glutamicum* ATCC13869 by homologous recombination on the chromosome to obtain a recombinant strain (van der Rest et al., Appl Microbiol Biotechnol 52:541-545, 1999). PCR was performed on the obtained recombinant strain using the primer pair of SEQ ID NOs: 14 and 17 to confirm that the deletion mutation was introduced into the argR gene on the chromosome. At this time, the PCR reaction was performed by repeating 30 cycles of denaturation at 95°C for 30 seconds; annealing at 55°C for 30 seconds; and extension at 72°C for 2 minutes. The transformant was named CJR1.

The argB(M54V) mutation was introduced into the *Corynebacterium glutamicum* CJR1 in the same manner as above. The prepared pDC24-argB(M54V) plasmid was used, and PCR was performed on the transformant in which the second recombination was completed using a primer pair (SEQ ID NOs: 18 and 21) to confirm that the M54V mutation was introduced into the argB gene on the chromosome, and the transformant was named *Corynebacterium glutamicum* CJR2 strain.

The sequences of the primers used in Comparative Example 1 are shown in Table 2 below.

**[TABLE 2]**

| Name | Sequence (5'-> 3') | SEQ ID NO |
|---|---|---|
| *argR*-5'-F | tgaattcgagctcggtaccccactggtgaactccttgtcc | SEQ ID NO: 14 |
| *argR*-5'-R | ttgaactaggggcgctttaaaagttttccggtgttgacgg | SEQ ID NO: 15 |
| *argR*-3'-F | ccgtcaacaccggaaaacttttaaagcgcccctagttcaa | SEQ ID NO: 16 |
| *argR*-3'-R | gtcgactctagaggatcccccgttgaactgcttgccagcc | SEQ ID NO: 17 |
| *argB*-5'-F | tgaattcgagctcggtaccctgcggctcgcacggttgctc | SEQ ID NO: 18 |
| *argB*-5'-R | acggtgcgcaagaagaccacgtcggcagcaaaagcagcct | SEQ ID NO: 19 |
| *argB*-3'-F | ggctgcttttgctgccgacgtggtcttcttgcgcaccgtg | SEQ ID NO: 20 |
| *argB*-3'-R | gtcgactctagaggatccccctcttatcaggccaatcggt | SEQ ID NO: 21 |

### Comparative Example 1-2. Preparation of a microorganism in which the argR gene is deleted, and an argB(M54V) gene mutation and argC gene enhancement are introduced

Using the *Corynebacterium glutamicum* CJR2 strain prepared in Comparative Example 1-1, a CJR100 strain, in which an N-acetyl-gamma-glutamyl-phosphate reductase (hereinafter, argC) gene was additionally enhanced and L-arginine productivity was improved, was prepared.

Specifically, to enhance the activity of the argC (SEQ ID NO: 23, NCBI Accession No. BBD29_RS07535), which is an N-acetyl-gamma-glutamyl-phosphate reductase gene, a plasmid for enhancing argC activity by replacing the wild-type promoter of the argC gene with Po2 was prepared using the o2 promoter (Korean Registered Patent No. 10-1632642, hereinafter Po2) known as a strong promoter. The upstream and downstream regions of the argC gene were obtained. More specifically, to prepare a strain into which argC with Po2 was introduced, PCR was performed using the chromosomal DNA of *Corynebacterium glutamicum* ATCC13869 as a template to amplify the gene fragment of the upstream region of the argC gene using primers of SEQ ID NO: 24 and SEQ ID NO: 25, and the gene fragment of the downstream region of the argC gene using primers of SEQ ID NO: 26 and SEQ ID NO: 27, respectively. PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and polymerization at 72°C for 1 minute were performed, and these denaturation, annealing, and polymerization steps were repeated 28 times. As a result, a 610 bp DNA fragment of the argC upstream region from *Corynebacterium glutamicum* ATCC13869 and a 1086 bp DNA fragment of the argC downstream region were obtained, respectively. PCR was performed using the o2 promoter (Korean Registered Patent No. 10-1632642) and the amplified argC upstream and downstream DNA fragments as templates, with the primers of SEQ ID NO: 24 and SEQ ID NO: 25. As PCR conditions, after initial denaturation at 95°C for 5 minutes, 28 cycles of denaturation at 95°C for 30 seconds; annealing at 55°C for 30 seconds; and polymerization at 72°C for 2 minutes were repeated, and then final polymerization was performed at 72°C for 5 minutes. Finally, a polymerization step was performed at 72°C for 5 minutes. The PCR fragment obtained above was subjected to DNA purification and then linked with a pDC24 plasmid treated with SmaI restriction enzyme using an In-Fusion^{®} HD cloning kit (Clontech) for fusion cloning. The resulting vector was named pDC24-Po2-argC.

Then, the *Corynebacterium glutamicum* CJR2 strain prepared in Comparative Example 1 was transformed with the prepared pDC24-Po2-argC plasmid by homologous recombination on the chromosome to obtain a recombinant strain (van der Rest et al., Appl Microbiol Biotechnol 52:541-545, 1999). PCR was performed on the obtained recombinant strain (also referred to as the transformant) using the primer pair of SEQ ID NOs: 27 and 28 to confirm that the argC gene on the chromosome was enhanced with Po2. At this time, the PCR reaction was performed by repeating 30 cycles of denaturation at 95°C for 30 seconds; annealing at 55°C for 30 seconds; and extension at 72°C for 2 minutes. The transformant was named *Corynebacterium glutamicum* CJR100 strain.

The sequences of the primers used in Comparative Example 2 are shown in Table 3 below.

**[TABLE 3]**

| Name | Sequence (5'-> 3') | SEQ ID NO |
|---|---|---|
| *argC*-5'-F | GTGAATTCGAGCTCGGTACCCGCCCCGAAAA GCCGTTAAAAG | SEQ ID NO: 24 |
| *argC*-5'-R | tgccaaaattcacgattattgCCCACCTACAGCTAAAACT GC | SEQ ID NO: 25 |
| *argC*-3'-F | ttattggaggagatcaaaacaATGACAATCAAGGTTGCA ATC | SEQ ID NO: 26 |
| *argC*-3'-R | TTAAGGTGCGACGCCGACCTGGGGGATCCTC TAGAGTCGACC | SEQ ID NO: 27 |
| Po2-F | caataatcgtgaattttggca | SEQ ID NO: 28 |
| Po2-R | tgttttgatctcctccaataa | SEQ ID NO: 29 |

### Example 2. Preparation of a glutamate-family amino acid-producing microorganism having enhanced fumarate hydratase activity and evaluation of glutamate-family amino acid production ability

### Example 2-1. Preparation of a glutamate-family amino acid-producing microorganism having enhanced fumarate hydratase activity

In order to prepare a glutamate-family amino acid microorganism having enhanced fumarate hydratase activity, the pDC24ΔBBD29_RS10775::Pspl13-fumC plasmid prepared in Example 1 was transformed into the *Corynebacterium glutamicum* CJR100 strain prepared in Comparative Example 1-2 by homologous recombination into the chromosome to obtain a recombinant strain (van der Rest et al., Appl Microbiol Biotechnol 52:541-545, 1999). PCR was performed on the obtained recombinant strain using the primer pair of SEQ ID NOs: 11 and 12 to confirm that fumarate hydratase was introduced in a form enhanced by Pspl13. The PCR reaction was performed by repeating 30 cycles of denaturation at 95°C for 30 seconds; annealing at 55°C for 30 seconds; and extension at 72°C for 2 minutes. The transformant was named *Corynebacterium glutamicum* CJR1002 (CJR100-Pspl13_fumC).

### Example 2-2. Evaluation of increase in glutamate-family amino acid production ability of a microorganism having enhanced fumarate hydratase activity

In order to confirm the effect of the enhancement of fumarate hydratase activity on glutamate-family amino acid production ability, the *Corynebacterium glutamicum* CJR1002 strain having enhanced fumarate hydratase activity prepared in Example 2-1, and the *Corynebacterium glutamicum* CJR2 strain and *Corynebacterium glutamicum* CJ100 strain prepared in Comparative Example 1-1 and Comparative Example 1-2, in which the activity of fumarate hydratase is non-enhanced, were cultured in the following manner, and the production ability of L-arginine, L-citrulline, and L-ornithine, which are glutamate-family amino acids, was confirmed.

Specifically, the *Corynebacterium glutamicum* CJR2 strain, *Corynebacterium glutamicum* CJ100 strain, and *Corynebacterium glutamicum* CJR1002 strain were each inoculated into a 250 ml corner-baffle flask containing 25 ml of the following production medium, and shaking culture was performed at 30°C for 44 hours at 200 rpm. The composition of the production medium is as follows.

### <Production medium (pH 7.2)>

Sucrose 50 g, ammonium sulfate 57 g, magnesium sulfate heptahydrate 2 g, beet molasses 5 g, calcium chloride 1 mg, cobalt chloride 1 mg, monopotassium phosphate 2 g, biotin 0.01 mg, thiamine-HCl 0.1 mg, calcium pantothenate 2 mg, nicotinamide 3 mg, ferrous sulfate 10 mg, manganese sulfate 10 mg, zinc sulfate 0.02 mg, copper sulfate 0.5 mg, calcium carbonate 30 g (based on 1 liter of distilled water)

After completion of cultivation, the production ability (concentration) of L-arginine, L-citrulline, and L-ornithine was measured using HPLC (Waters 2478). The experiment was repeated three times, and the average concentration values of L-arginine, L-citrulline, and L-ornithine of the analysis results are shown in Table 4 below.

**[TABLE 4]**

| Strain name | L-arginine (g/ℓ) | L-citrulline (g/ℓ) | L-ornithine (g/ℓ) |
|---|---|---|---|
| CJR2 | 5.1 | 1.0 | 0.1 |
| CJR100 | 5.9 | 1.0 | 0.2 |
| CJR1002(CJR100-Pspl13_*fumC*) | 6.2 | 1.3 | 0.4 |

As a result, as shown in Table 4, it was confirmed that the *Corynebacterium glutamicum* CJR1002 strain in which the fumC gene was enhanced with Pspl13, which is a strong promoter, had improved L-arginine production ability compared to the non-enhanced strain, Corynebacterium glutamicum CJR100, showing an L-arginine production ability of 105%, and L-citrulline and L-ornithine production abilities of 130% and 200%, respectively.

From the above results, it was confirmed that the enhanced activity of fumarate hydratase increases the glutamate-family amino acid production ability of microorganisms of the genus *Corynebacterium*.

### Example 3. Construction of a plasmid for inserting a gene encoding a complex comprising malate:quinone oxidoreductase, malate dehydrogenase, aspartate transaminase, NADP-specific glutamate dehydrogenase, and fumarate hydratase

In order to confirm the productivity improvement effect of glutamate-family amino acids according to the introduction of a complex comprising all of the following genes: the gene (SEQ ID NO: 31, hereinafter mqo) encoding malate:quinone oxidoreductase (SEQ ID NO: 30) of *Corynebacterium glutamicum*; the gene (SEQ ID NO: 35, hereinafter mdh) encoding malate dehydrogenase (SEQ ID NO: 34); the gene (SEQ ID NO: 37, hereinafter aspB) encoding aspartate transaminase (SEQ ID NO: 36); the gene (SEQ ID NO: 39, hereinafter gdh) encoding NADP-specific glutamate dehydrogenase (SEQ ID NO: 38); and the gene (SEQ ID NO: 41, hereinafter fumC) encoding fumarate hydratase (SEQ ID NO: 40), a complex enhancement vector was prepared in the following manner.

Specifically, in order to insert the gene complex into the *Corynebacterium glutamicum* chromosome, the mqo gene in the complex was used as an insertion site. In order to substitute the sequence within the mqo gene with the complex form, a target gene insertion vector comprising the mqo gene was prepared. Using the chromosome of the *Corynebacterium glutamicum* ATCC13032 strain as a template, PCR was performed using primer pairs for each of the genes in the complex listed in Table 5.

PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and polymerization at 72°C for 1 minute were performed, and denaturation, annealing, and polymerization under these conditions were repeated 28 times. As a result, the following DNA fragments were obtained: a 1947 bp mqo gene fragment using the primer pair of SEQ ID NOs: 42 and 43; a 1357 bp mdh gene fragment using the primer pair of SEQ ID NOs: 44 and 45; a 1575 bp aspB gene fragment using the primer pair of SEQ ID NOs: 46 and 47; a 1794 bp gdh gene fragment using the primer pair of SEQ ID NOs: 48 and 49; a 1686 bp fumC gene fragment using the primer pair of SEQ ID NOs: 50 and 51; and a 1916 bp mqo gene fragment using the primer pair of SEQ ID NOs: 52 and 53. PCR was performed using the obtained DNA products with the primers of SEQ ID NO: 42 and SEQ ID NO: 53. PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and polymerization at 72°C for 10 minutes were performed, and denaturation, annealing, and polymerization under these conditions were repeated 28 times. As a result, a 10275 bp mqo-mdh-aspB-gdh-fumC-mqo complex gene DNA fragment was obtained. The obtained DNA product was purified using a PCR purification kit (PCR Purification kit, QUIAGEN), and the purified amplification product was treated with the restriction enzyme SmaI, and then, using the pDC24 vector (SEQ ID NO: 146) heat-treated at 65°C for 20 minutes and the In-Fusion cloning kit (Infusion Cloning Kit, TaKaRa), a pDC24 mqo-mdh-aspB-gdh-fumC-mqo vector for introducing mqo-mdh-aspB-gdh-fumC-mqo into the chromosome was prepared according to the provided manual.

The sequences of the primers used in Example 3 are shown in Table 5 below.

**[TABLE 5]**

| Name | Sequence (5'-> 3') | SEQ ID NO |
|---|---|---|
| *mqo*-5'-F | GTGAATTCGAGCTCGGTACCCATACTACTCATGT TTGCGAAT | SEQ ID NO: 42 |
| *mqo*-3'-R | TCCTGATATCGGGCACCATTTGGCAAAGAATACG CAAAGCAC | SEQ ID NO: 43 |
| *mdh*-5'-F | TGCTTTGCGTATTCTTTGCCAAATGGTGCCCGAT ATCAGGA | SEQ ID NO: 44 |
| *mdh*-3'-R | AACAAAGCAGCCATGCGTTGCAGCCGTTACTTAA ACCAAGTC | SEQ ID NO: 45 |
| *aspB*-5'-F | CGACTTGGTTTAAGTAACGGCTGCAACGCATGGC TGCTTTGTT | SEQ ID NO: 46 |
| *aspB*-3'-R | GCGAATGAGACCAGTTGACTGTTAGTTAGCGTAA TGCTCCGC | SEQ ID NO: 47 |
| *gdh*-5'-F | GCGGAGCATTACGCTAACTAACAGTCAACTGGTC TCATTCGC | SEQ ID NO: 48 |
| *gdh*-3'-R | GGTTAATATCCAATATGGAAGTTAGATGACGCCC TGTGCCAG | SEQ ID NO: 49 |
| *fumC*-5'-F | CTGGCACAGGGCGTCATCTAACTTCCATATTGGA TATTAACC | SEQ ID NO: 50 |
| *fumC*-3'-R | ATTCGCAAACATGAGTAGTATTTAGAACTTGTTC TCGCGCTC | SEQ ID NO: 51 |
| *mqo*-5'-F | CGCGAGAACAAGTTCTAAATACTACTCATGTTTG CGAATTG | SEQ ID NO: 52 |
| *mqo*-3'-R | GGTCGACTCTAGAGGATCCCCTTAGGCTTCCTCA AGCTTCAG | SEQ ID NO: 53 |
| Mqo-F | CTCTTCACCAGCATT | SEQ ID NO: 54 |
| Mqo-R | TTGTCACAACATCTGTTTCA | SEQ ID NO: 55 |

### Example 4. Preparation of a microorganism in which the activities of malate:quinone oxidoreductase, malate dehydrogenase, aspartate transaminase, NADP-specific glutamate dehydrogenase, and fumarate hydratase are all enhanced, and evaluation of glutamate-family amino acid production ability

### Example 4-1. Preparation of a microorganism in which the activities of malate:quinone oxidoreductase, malate dehydrogenase, aspartate transaminase, NADP-specific glutamate dehydrogenase, and fumarate hydratase are all enhanced

In order to prepare a microorganism in which the activities of malate:quinone oxidoreductase, malate dehydrogenase, aspartate transaminase, NADP-specific glutamate dehydrogenase, and the activity of fumarate hydratase are all enhanced, the pDC24 mqo-mdh-aspB-gdh-fumC-mqo plasmid prepared in Example 3 was transformed into the *Corynebacterium glutamicum* CJR100 strain prepared in Comparative Example 1-2 by homologous recombination into the chromosome to obtain a recombinant strain (van der Rest et al., Appl Microbiol Biotechnol 52:541-545, 1999). PCR was performed on the obtained recombinant strain using the primer pair of SEQ ID NOs: 54 and 55 to confirm that the mqo-mdh-aspB-gdh-fumC-mqo complex was introduced into the *Corynebacterium glutamicum* chromosome. The PCR reaction was performed by repeating 30 cycles of denaturation at 95°C for 30 seconds; annealing at 55°C for 30 seconds; and extension at 72°C for 2 minutes. The transformant was named *Corynebacterium glutamicum* CJR1012 strain.

### Example 4-2. Confirmation of an increase in glutamate-family amino acid production ability of a microorganism in which the activities of malate:quinone oxidoreductase, malate dehydrogenase, aspartate transaminase, NADP-specific glutamate dehydrogenase, and fumarate hydratase are all enhanced

In order to confirm the effect of the enhancement of the activities of malate:quinone oxidoreductase, malate dehydrogenase, aspartate transaminase, NADP-specific glutamate dehydrogenase, and fumarate hydratase on L-amino acid production ability, the *Corynebacterium glutamicum* CJR1012 strain prepared in Example 4-1, the *Corynebacterium glutamicum* CJR1002 strain prepared in Example 2-1 in which only fumarate hydratase activity was enhanced, and the *Corynebacterium glutamicum* CJR2 strain and *Corynebacterium glutamicum* CJ100 strain prepared in Comparative Example 1-1 and Comparative Example 1-2 in which fumarate hydratase activity was not enhanced, were cultured in the following manner to confirm the production ability of L-arginine.

Specifically, the *Corynebacterium glutamicum* CJR2 strain, the *Corynebacterium glutamicum* CJ100 strain, the *Corynebacterium glutamicum* CJR1002 strain, and the *Corynebacterium glutamicum* CJR1012 strain were respectively inoculated into a 250 ml corner-baffle flask containing 25 ml of the following production medium and shaking culture was performed at 30°C for 44 hours at 200 rpm. The composition of the production medium is as follows.

### <Production medium (pH 7.2)>

Sucrose 50 g, ammonium sulfate 57 g, magnesium sulfate heptahydrate 2 g, beet molasses 5 g, calcium chloride 1 mg, cobalt chloride 1 mg, monopotassium phosphate 2 g, biotin 0.01 mg, thiamine-HCl 0.1 mg, calcium pantothenate 2 mg, nicotinamide 3 mg, ferrous sulfate 10 mg, manganese sulfate 10 mg, zinc sulfate 0.02 mg, copper sulfate 0.5 mg, calcium carbonate 30 g (based on 1 liter of distilled water)

After completion of cultivation, the production ability (concentration) of L-arginine was measured using HPLC (Waters 2478). The experiment was repeated three times, and the average value of the L-arginine concentration, which is the result of the analysis, is shown in Table 6 below.

**[TABLE 6]**

| Strain name | L- arginine (g/ℓ) |
|---|---|
| CJR2 | 5.1 |
| CJR100 | 5.9 |
| CJR1002(CJR100-Pspl13_*fumC*) | 6.2 |
| CJR1012(CJR100-*mqo*-*mdh*-*aspB*-*gdh*-*fumC-mqo*) | 6.4 |

As a result, as shown in Table 6, it was confirmed that the *Corynebacterium glutamicum* CJR1012 strain, into which pDC24 mqo-mdh-aspB-gdh-fumC-mqo was introduced, exhibited an L-arginine production ability of 109% compared to the *Corynebacterium glutamicum* CJR100 strain, in which the fumC gene is not enhanced, and it was also confirmed that its L-arginine production ability was higher than that of the CJR1002 strain in which the fumC gene alone was enhanced.

From the above results, it was confirmed that the enhancement of the activities of malate:quinone oxidoreductase, malate dehydrogenase, aspartate transaminase, NADP-specific glutamate dehydrogenase, and fumarate hydratase increased the production ability of arginine as a representative example among the glutamate-family amino acids, thereby increasing the glutamate-family amino acid production ability of microorganisms of the genus *Corynebacterium*.

### Example 5. Preparation of an O-acetyl homoserine-producing microorganism having enhanced fumarate hydratase activity and evaluation of O-acetyl homoserine production ability

### Example 5-1. Construction of a plasmid for the insertion of a gene encoding fumarate hydratase

In order to confirm the effect of the enhancement of the activity of fumarate hydratase on the production of O-acetyl homoserine in a *Corynebacterium glutamicum* strain having O-acetyl homoserine and L-homoserine production ability, a vector for introducing a gene (SEQ ID NO: 41, NCBI Accession No. "NCgl0967", hereinafter fumC) encoding fumarate hydratase derived from *Corynebacterium glutamicum* ATCC13032 into the *Corynebacterium glutamicum* O-acetyl homoserine and homoserine-producing strain ATCC13032 ΔNCgl2335::PCJ7-yjeH(eco,F351L) ΔmetB ΔmetY lysC(L377K)ΔNCgl0616 (Korean Patent Publication No. 10-2022-0038131) was prepared as follows.

Specifically, in order to insert the fumC, which is a fumarate hydratase gene, into the *Corynebacterium glutamicum* chromosome, the NCgl1490 gene, which is known as a gene encoding a transposon in *Corynebacterium glutamicum*, was used as an insertion site (site) (Journal of Biotechnology 104, 5-25 Jorn Kalinowski et al, 2003). In order to substitute the NCgl1490 gene with the fumC enhancement form, an NCgl1490 deletion and target gene insertion vector was prepared. In order to prepare the vector, PCR was performed using the primer pairs of SEQ ID NOs: 58 and 59, and SEQ ID NOs: 60 and 61 using the chromosome of the *Corynebacterium glutamicum* ATCC13032 strain as a template.

PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and polymerization at 72°C for 1 minute were performed, and denaturation, annealing, and polymerization under these conditions were repeated 28 times. As a result, a 1057 bp DNA fragment of the NCgl1490 upstream region and an 1146 bp DNA fragment of the NCgl1490 downstream region from *Corynebacterium glutamicum* ATC13032 were respectively obtained. PCR was performed with the primers of SEQ ID NO: 58 and SEQ ID NO: 61, using the amplified promoter and DNA fragments as templates. As PCR conditions, after denaturation at 95°C for 5 minutes, 28 cycles of denaturation at 95°C for 30 seconds; annealing at 55°C for 30 seconds; and polymerization at 72°C for 2 minutes were repeated, and then final polymerization was performed at 72°C for 5 minutes. The two fragments obtained above were subjected to DNA purification, and then a plasmid was obtained by fusion cloning using an In-Fusion^{®} HD cloning kit (Clontech) and the pDC24 vector (SEQ ID NO: 146) treated with SmaI restriction enzyme according to the provided manual. The obtained vector was named pDC24-ΔNCgl1490.

In order to enhance the activity of fumarate hydratase, a plasmid for enhancing the fumC gene was prepared using the Pspl13 promoter (SEQ ID NO: 9, Korean Registered Patent No. 10-1783170), which is known as a strong promoter.

Specifically, in order to prepare a gene into which the FumC having Pspl13 was introduced, PCR was performed on the fumC gene fragment using the chromosome of *Corynebacterium glutamicum* ATCC13032 as a template and using the primers of SEQ ID NO: 62 and SEQ ID NO: 63, respectively. In addition, the Pspl13 promoter fragment was obtained using pDC24-Pspl13 as a template and using the primers of SEQ ID NO: 64 and SEQ ID NO: 65.

PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and polymerization at 72°C for 1 minute were performed, and denaturation, annealing, and polymerization under these conditions were repeated 28 times. As a result, a 330 bp DNA fragment of the Pspl13 promoter region and a 1448 bp DNA fragment of fumC derived from *Corynebacterium glutamicum* ATC13032 were respectively obtained. PCR was performed using the primers of SEQ ID NO: 64 and SEQ ID NO: 62 using the amplified promoter and DNA fragment as templates. As PCR conditions, after initial denaturation at 95°C for 5 minutes, 28 cycles of denaturation at 95°C for 30 seconds; annealing at 55°C for 30 seconds; and polymerization at 72°C for 2 minutes were repeated, and then final polymerization was performed at 72°C for 5 minutes.

As a result, a 1738 bp Pspl13_fumC DNA fragment, which has the Pspl13 promoter and encodes FumC, was amplified. The amplification product was purified using a PCR purification kit (PCR Purification kit, QIAGEN) and used as an insert DNA fragment for the preparation of the vector. After the purified amplification product was treated with the restriction enzyme SmaI, the molar concentration (M) ratio of the vector pDC24ΔNCgl1490, which had been heat-treated at 65°C for 20 minutes, to the insert DNA fragment, which is the amplification product, was set to 1:2, and by performing cloning using the In-Fusion^{®} HD cloning kit (Clontech), which is an In-Fusion cloning kit, according to the provided manual, the vector pDC24-ΔNCgl1490:Pspl13-fumC for introducing Pspl13-fumC into the chromosome was prepared..

The sequences of the primers used in Example 5-1 are shown in Table 7 below.

**[TABLE 7]**

| Name | Sequence (5'-> 3') | SEQ ID NO |
|---|---|---|
| *NCgl1490*-up-F | AGTGAATTCGAGCTCGGTACCCGCGACATTT CGATGACCTAGGA | SEQ ID NO: 58 |
| *NCgl1490*-up-R | AGAGTGGCAGCAGTCCCGGGAGGGAAATTA GTCAGA | SEQ ID NO: 59 |
| *NCgl1490*-down-F | TGACTAATTTCCCTCCCGGGACTGCTGCCACT CTCACTGCTT | SEQ ID NO: 60 |
| *NCgl1490*-down-R | GGTCGACTCTAGAGGATCCCCTACAGTGCTT CAGTATCGGTA | SEQ ID NO: 61 |
| *fumC*-3'-F | TTATTGGAGGAGATCAAAACAATGACCGAGC AGGAATTCC | SEQ ID NO: 62 |
| *fumC*-3'-R | AGAGTGGCAGCAGTCCCTTAGAACTTGTTCT CGCGCT | SEQ ID NO: 63 |
| Pspl13-F | TGACTAATTTCCCTCCCGGCGCTTCATGTCAA CAATC | SEQ ID NO: 64 |
| Pspl13-R | GGAATTCCTGCTCGGTCATTGTTTTGATCTCC TCCAATA | SEQ ID NO: 65 |

### Example 5-2. Preparation of an O-acetyl homoserine-producing microorganism having enhanced fumarate hydratase activity

In order to prepare an O-acetyl homoserine-producing microorganism having enhanced fumarate hydratase activity, the pDC24-ΔNCgl1490:Pspl13-fumC plasmid prepared in Example 5-1 was introduced into the *Corynebacterium glutamicum* O-acetyl homoserine and homoserine-producing strain ATCC13032 ΔNCgl2335::PCJ7-yjeH(eco,F351L) ΔmetB ΔmetY lysC(L377K)ΔNCgl0616 (Korean Patent Publication No. 10-2022-0038131) by homologous recombination on the chromosome to obtain a recombinant strain (van der Rest et al., Appl Microbiol Biotechnol 52:541-545, 1999). PCR was performed on the obtained recombinant strain using the primer pair of SEQ ID NOs: 58 and 63 to confirm that the fumarate hydratase gene was introduced into the chromosome in a form enhanced by Pspl13. The PCR reaction was performed by repeating 30 cycles of denaturation at 95°C for 30 seconds; annealing at 55°C for 30 seconds; and extension at 72°C for 2 minutes. The transformant was named *Corynebacterium glutamicum* CM04-8005 strain.

### Example 5-3. Preparation of an O-acetyl homoserine-producing microorganism in which the activities of malate:quinone oxidoreductase, malate dehydrogenase, aspartate transaminase, NADP-specific glutamate dehydrogenase, and fumarate hydratase are simultaneously enhanced

In order to prepare an O-acetyl homoserine-producing microorganism in which the activities of malate:quinone oxidoreductase, malate dehydrogenase, aspartate transaminase, NADP-specific glutamate dehydrogenase, and fumarate hydratase are all enhanced, the pDC24Δ mqo-mdh-aspB-gdh-fumC-mqo plasmid prepared in Example 3 was transformed into the *Corynebacterium glutamicum* O-acetyl homoserine and homoserine-producing strain ATCC13032 ΔNCgl2335::PCJ7-yjeH(eco,F351L) ΔmetB ΔmetY lysC(L377K)ΔNCgl0616 (Korean Patent Publication No. 10-2022-0038131) by homologous recombination on the chromosome to obtain a recombinant strain (van der Rest et al., Appl Microbiol Biotechnol 52:541-545, 1999). PCR was performed on the obtained recombinant strain using the primer pair of SEQ ID NOs: 54 and 55 to confirm that the mqo-mdh-aspB-gdh-fumC-mqo complex was introduced into the chromosome. The PCR reaction was performed by repeating 30 cycles of denaturation at 95°C for 30 seconds; annealing at 55°C for 30 seconds; and extension at 72°C for 2 minutes. The transformant was named *Corynebacterium glutamicum* CM04-8006 strain.

### Example 5-4. Confirmation of increase in O-acetyl homoserine production ability of an O-acetyl homoserine-producing microorganism having enhanced fumarate hydratase activity and an O-acetyl homoserine-producing microorganism in which the activities of malate:quinone oxidoreductase, malate dehydrogenase, aspartate transaminase, NADP-specific glutamate dehydrogenase, and fumarate hydratase are simultaneously enhanced

In order to confirm the increase in the O-acetyl homoserine production ability of an O-acetyl homoserine-producing microorganism in which the enhancement of fumarate hydratase activity and the activities of malate:quinone oxidoreductase, malate dehydrogenase, aspartate transaminase, and NADP-specific glutamate dehydrogenase, and fumarate hydratase are simultaneously enhanced, the concentration of O-acetyl homoserine produced after culture was measured in the following manner.

Specifically, the control group *Corynebacterium glutamicum* ATCC13032 ΔNCgl2335::PCJ7-yjeH(eco,F351L) ΔmetB ΔmetY lysC(L377K)ΔNCgl0616, the *Corynebacterium glutamicum* CM04-8005 strain prepared in Example 5-2, and the *Corynebacterium glutamicum* CM04-8006 strain prepared in Example 5-3 were each inoculated with one inoculation loop into a 250 ml corner-baffle flask containing 25 ml of the following O-acetyl homoserine production medium and subjected to shaking culture at 33°C for 20 hours at 200 rpm. The composition of the production medium is as follows.

### <O-acetyl homoserine production medium (pH 7.2)>

Glucose 30 g, KH₂PO₄ 2 g, urea 3 g, (NH₄)₂SO₄ 40 g, peptone 2.5 g, CSL(Sigma) 5 g(10 ml), MgSO₄.7H₂O 0.5 g, CaCO₃ 20 g (based on 1 liter of distilled water)

After the cultivation was completed, the O-acetyl homoserine production ability (concentration) was measured using HPLC (Waters 2478). The experiment was repeated three times, and the average concentration value of O-acetyl homoserine as the analysis results is shown in Table 8 below.

**[TABLE 8]**

| Strain name | O-acetyl homoserine (g/L) |
|---|---|
| ATCC13032 | 0.27 |
| ATCC13032Δ*NCgl2335*::PCJ7-*yjeH*(eco,F351L) Δ*metB* Δ*metY lysC*(L377K)Δ*NCgl0616* | 1.73 |
| CM04-8005 (ATCC13032 Δ*NCgl2335*::PCJ7-*yjeH*(eco,F351L) Δ*metB* Δ*metY lysC*(L377K)Δ*NCgl0616* Δ*NCgl1490*::Pspl13_*fumC*) | 2.21 |
| CM04-8006 (ATCC13032 Δ*NCgl2335*::PCJ7- | 2.32 |
| *yjeH*(eco,F351L) Δ*metB* Δ*metY lysC*(L377K)Δ*NCgl0616*_ *mqo*-*mdh*-*aspB*-*gdh*-*fumC*-*mqo*) | |

As a result, as shown in Table 8, compared to the *Corynebacterium glutamicum* ATCC13032 ΔNCgl2335::PCJ7-yjeH(eco,F351L) ΔmetB ΔmetY lysC(L377K)ΔNCgl0616 strain in which the activity of fumarate hydratase was non-enhanced, it was confirmed that the O-acetyl homoserine concentration in the *Corynebacterium glutamicum* CM04-8005 strain, having enhanced fumarate hydratase activity, was approximately 128%, and the O-acetyl homoserine concentration in the *Corynebacterium glutamicum* CM04-8006 strain, in which the activities of malate:quinone oxidoreductase, malate dehydrogenase, aspartate transaminase, NADP-specific glutamate dehydrogenase, and fumarate hydratase were simultaneously enhanced, was approximately 134%.

From the above results, it was confirmed that the enhancement of the activity of fumarate hydratase and/or the simultaneous enhancement of the activities of malate:quinone oxidoreductase, malate dehydrogenase, aspartate transaminase, NADP-specific glutamate dehydrogenase, and fumarate hydratase increases the O-acetyl homoserine production ability of microorganisms of the genus *Corynebacterium*.

### Example 5-5. Confirmation of increase in O-acetyl homoserine production ability of an O-acetyl homoserine-producing microorganism having additionally enhanced O-acetyl homoserine transferase activity and enhanced fumarate hydratase activity; and an O-acetyl homoserine-producing microorganism having additionally enhanced O-acetyl homoserine transferase activity and simultaneously enhanced activities of malate:quinone oxidoreductase, malate dehydrogenase, aspartate transaminase, NADP-specific glutamate dehydrogenase, and fumarate hydratase

In an O-acetyl homoserine-producing microorganism in which the activity of O-acetyl homoserine transferase was additionally enhanced, the following experiment was performed to confirm the effect of increasing the O-acetyl homoserine production ability according to the enhancement of the activity of fumarate hydratase and the simultaneous enhancement of the activities of malate:quinone oxidoreductase, malate dehydrogenase, aspartate transaminase, NADP-specific glutamate dehydrogenase, and fumarate hydratase. Specifically, in order to amplify the metX gene encoding O-acetyl homoserine transferase, nucleotide sequence information (SEQ ID NO: 67, NCBI Accession No. NCgl0624) of the metX gene was secured from the NIH GenBank of the National Institutes of Health, and based on this, BamHI restriction enzyme sites were inserted at both ends of the primers of SEQ ID NOs: 68 and 69 for amplifying a region covering from the promoter region (about 300 bp upstream of the start codon) to the terminator region (about 100 bp downstream of the stop codon). As PCR conditions, after initial denaturation at 95°C for 5 minutes, 30 cycles of denaturation at 95°C for 30 seconds, annealing at 55°C for 30 seconds, and polymerization at 72°C for 90 seconds were repeated, and then a polymerization reaction was performed at 72°C for 7 minutes. As a result, a 1546 bp DNA fragment corresponding to the coding region of the metX gene was obtained. The pECCG117 (Korean Registered Patent No. 10-0057684) vector and the metX DNA fragment were treated with the restriction enzyme BamHI, connected using DNA ligase, and then cloned to obtain a plasmid, which was named pECCG117-metX WT.

The primer sequences used in Example 5-5 are shown in Table 9 below.

**[TABLE 9]**

| Name | Sequence (5'-> 3') | SEQ ID NO |
|---|---|---|
| *metX* F | GGATCCCCTCGTTGTTCACCCAGCAACC | SEQ ID NO: 68 |
| *metX* R | GGATCCCAAAGTCACAACTACTTATGTTA G | SEQ ID NO: 69 |

The prepared pECCG117-metX WT vector was introduced into the *Corynebacterium glutamicum* ATCC13032 ΔNCgl2335::PCJ7-yjeH(eco,F351L) ΔmetB ΔmetY lysC(L377K)ΔNCgl0616, the *Corynebacterium glutamicum* CM04-8005 strain prepared in Example 5-2, and the *Corynebacterium glutamicum* CM04-8006 strain prepared in Example 5-3 by electroporation, and then spread on a selection medium containing 25 mg/L of kanamycin to obtain each transformant.

In order to compare the O-acetyl homoserine production ability of the strains prepared above, the strains were cultured in the following manner to analyze the O-acetyl homoserine concentration in the culture broth.

The strains were inoculated with one inoculation loop into a 250 ml corner-baffle flask containing 25 ml of the following O-acetyl homoserine production medium, and subjected to shaking culture at 33°C for 20 hours at 200 rpm. Then, the O-acetyl homoserine concentration was analyzed using HPLC, and the analyzed concentration values are shown in Table 10 below.

### <O-acetyl homoserine production medium (pH 7.2)>

Glucose 30 g, KH₂PO₄ 2 g, urea 3 g, (NH₄)₂SO₄ 40 g, peptone 2.5 g, CSL(Sigma) 5 g(10 ml), MgSO₄.7H₂O 0.5 g, CaCO₃ 20 g (based on 1 liter of distilled water)

**[TABLE 10]**

| Name | O-acetyl homoserine (g/L) |
|---|---|
| ATC*C*13032Δ*NCgl2335*::P*C*J7-*yjeH*(eco,F351L) Δ*metB* Δ*metY lysC*(L377K)Δ*NCgl0616* | 1.73 |
| CM04-8004 (ATCC13032 Δ*NCgl2335*::PCJ7-*yjeH*(eco,F35*1*L) Δ*metB* Δ*metY*_ *lysC*(L377K)Δ*NCgl0616* Δ*NCgl1490*) | 1.76 |
| CM04-8005 (ATCC13032 Δ*NCgl2335*::PCJ7-*yjeH*(eco,F351L) Δ*metB*Δ*metY*_*lysC*(L377K)Δ*NCgl0616*Δ*NCgl1490*::Psp l13_*fumC*) | 2.21 |
| CM04-8006 (ATCC13032 Δ*NCgl2335*::PCJ7-*yjeH*(eco,F351L) Δ*metB* | 2.32 |
| Δ*metY*_*lysC*(L377K)Δ*NCgl0616*_ *mqo*-*mdh*-*aspB*-*gdh-fumC*-*mqo*) | |
| ATCC13032Δ*NCgl2335*::PCJ7-*yjeH*(eco,F351L) Δ*metB* Δ*metY*_*lysC*(L377K)Δ*NCgl0616*) **/pECCG117-*metX* WT** | 2.83 |
| CM04-8004 (ATCC13032 Δ*NCgl2335*::PCJ7-*yjeH*(*e*co,F351L) Δ*metB*Δ*metY*_*lysC*(L377K)Δ*NCgl0616*Δ*NCgl1490*)) **/pECCG117-*metX* WT** | 2.87 |
| CM04-8005 (ATCC13032 Δ*NCgl2335*::PCJ7-*yjeH*(eco,F351L) Δ*metB*Δ*metY*_*lysC*(L377K)Δ*NCgl0616*Δ*NCgl1490*::Psp l13_*fumC*)**/pECCG117-*metX* WT** | 4.22 |
| CM04-8006 (ATCC13032 Δ*NCgl2335*::PCJ7-*yjeH*(eco,F351L) Δ*metB*Δ*metY*_*lysC*(L3**77**K)Δ*N**C**gl0616*_***m**qo*-*mdh-aspB*-*gdh*-*fumC*-*mqo*) )**/pECCG117-*metX* WT** | 4.57 |

As a result, as shown in Table 10, when the ATCC13032 ΔNCgl2335::PCJ7-yjeH(eco,F351L) ΔmetB ΔmetY lysC(L377K) ΔNCgl0616/pECCG117-metX WT strain was cultured, the concentration of O-acetyl homoserine was 2.83 g/L, confirming an increase compared to 1.76 g/L, the O-acetyl homoserine concentration of the CM04-8004 (ATCC13032 ΔNCgl2335::PCJ7-yjeH(eco,F351L) ΔmetB ΔmetY_lysC(L377K)ΔNCgl0616 ΔNCgl1490) strain, which is a strain prior to enhancement of the metX gene. In addition, when the CM04-8005 strain in which the metX gene was additionally enhanced was cultured, the concentration of O-acetyl homoserine was 4.22 g/L, confirming that the productivity of O-acetyl homoserine was 149% compared to a strain in which the metX gene was enhanced but the activity of fumarate hydratase was not enhanced. Furthermore, when the CM04-8006 strain in which the metX gene was additionally enhanced was cultured, the concentration of O-acetyl homoserine was 4.57 g/L, confirming that the productivity of O-acetyl homoserine was approximately 161% compared to a strain in which the metX gene was enhanced but the activities of malate:quinone oxidoreductase, malate dehydrogenase, aspartate transaminase, NADP-specific glutamate dehydrogenase, and fumarate hydratase were not enhanced.

From the above results, it was confirmed that in a strain in which the O-acetyl-L-homoserine production ability was increased by metX gene enhancement, the production ability of O-acetyl-L-homoserine was increased upon enhancement of fumarate hydratase activity and upon simultaneous enhancement of the activities of malate:quinone oxidoreductase, malate dehydrogenase, aspartate transaminase, NADP-specific glutamate dehydrogenase, and fumarate hydratase.

### Example 6. Preparation of a homoserine-producing microorganism having enhanced fumarate hydratase activity and evaluation of homoserine production ability

### Example 6-1. Preparation of homoserine-producing microorganism having enhanced fumarate hydratase activity

In order to confirm the effect of enhancement of fumarate hydratase activity on homoserine production in a *Corynebacterium glutamicum* strain having homoserine production ability, the pDC24-ΔNCgl1490:Pspl13-fumC plasmid prepared in Example 5-1 was transformed into the *Corynebacterium glutamicum* KCCM12120P strain, which is a known homoserine-producing strain (Korean Registered Patent No. 10-1947959), to obtain a recombinant strain (van der Rest et al., Appl Microbiol Biotechnol 52:541-545, 1999). PCR was performed on the obtained recombinant strain using the primer pair of SEQ ID NOs: 58 and 62 to confirm that fumarate hydratase was introduced into the chromosome in a form enhanced by Pspl13. The PCR reaction was performed by repeating 30 cycles of: denaturation at 95°C for 30 seconds; annealing at 55°C for 30 seconds; and extension at 72°C for 2 minutes. The transformant was named *Corynebacterium glutamicum* CM10-0194 strain.

### Example 6-2. Evaluation of increase in homoserine production ability of a homoserine-producing microorganism having enhanced fumarate hydratase activity

In order to confirm the increase in the homoserine production ability of a homoserine-producing microorganism in which the activity of fumarate hydratase is enhanced, the strains were cultured in the following manner and the L-homoserine concentration in the culture broth was analyzed.

Specifically, the control group *Corynebacterium glutamicum* KCCM12120P strain and the *Corynebacterium glutamicum* CM10-0194 strain prepared in Example 6-1 were each inoculated with one inoculation loop into a 250 ml corner-baffle flask containing 25 ml of the following homoserine production medium and subjected to shaking culture at 33°C for 20 hours at 200 rpm.

### <Homoserine production medium (pH 7.2)>

### Glucose 30 g, KH₂PO₄ 2 g, urea 3 g, (NH₄)₂SO₄ 40 g, peptone 2.5 g, CSL(Sigma) 5 g(10 ml), MgSO₄.7H₂O 0.5 g, CaCO₃ 20 g (based on 1 liter of distilled water)

After the cultivation was completed, homoserine production ability (concentration) was measured using HPLC (Waters 2478). The experiment was repeated three times, and the average concentration value of homoserine as the analysis results is shown in Table 11 below.

**[TABLE 11]**

| Strain name | homoserine (g/L) |
|---|---|
| KCCM12120P | 0.95 |
| CM10-0194(KCCM12120PΔ*NCgl1490*:Pspl13-*fumC*) | 1.12 |

As a result, as shown in Table 11, it was confirmed that the concentration of homoserine in the culture broth of the *Corynebacterium glutamicum* CM10-0194 strain, in which the activity of fumarate hydratase was enhanced, was approximately 118% compared to the *Corynebacterium glutamicum* KCCM12120P strain, a homoserine-producing strain in which the activity of fumarate hydratase was non-enhanced.

### Example 6-3. Preparation of a homoserine-producing microorganism in which the activities of malate:quinone oxidoreductase, malate dehydrogenase, aspartate transaminase, NADP-specific glutamate dehydrogenase, and fumarate hydratase are simultaneously enhanced

In order to prepare a homoserine-producing microorganism in which the activities of malate:quinone oxidoreductase, malate dehydrogenase, aspartate transaminase, NADP-specific glutamate dehydrogenase, and the activity of fumarate hydratase are all enhanced, the pDC24Δ mqo-mdh-aspB-gdh-fumC-mqo plasmid prepared in Example 3 was transformed into the *Corynebacterium glutamicum* KCCM12120P strain, which is a known homoserine-producing strain (Korean Registered Patent No. 10-1947959), to obtain a recombinant strain (van der Rest et al., Appl Microbiol Biotechnol 52:541-545, 1999). PCR was performed on the obtained recombinant strain using the primer pair of SEQ ID NOs: 54 and 55 to confirm that the mqo-mdh-aspB-gdh-fumC-mqo complex was introduced into the chromosome. The PCR reaction was performed by repeating 30 cycles of: denaturation at 95°C for 30 seconds; annealing at 55°C for 30 seconds; and extension at 72°C for 2 minutes. The transformant was named *Corynebacterium glutamicum* CM10-0195 strain.

### Example 6-4. Evaluation of increase in homoserine production ability of a homoserine-producing microorganism in which the activities of malate:quinone oxidoreductase, malate dehydrogenase, aspartate transaminase, NADP-specific glutamate dehydrogenase, and fumarate hydratase are simultaneously enhanced

In order to confirm the increase in the homoserine production ability of a homoserine-producing microorganism in which the activity of fumarate hydratase is enhanced and the activities of malate:quinone oxidoreductase, malate dehydrogenase, aspartate transaminase, NADP-specific glutamate dehydrogenase, and the activity of fumarate hydratase are simultaneously enhanced, the concentration of homoserine produced after culture was measured in the following manner.

Specifically, the control group *Corynebacterium glutamicum* KCCM12120P strain and the *Corynebacterium glutamicum* CM10-0195 strain prepared in Example 6-3 were each inoculated with one inoculation loop into a 250 ml corner-baffle flask containing 25 ml of the homoserine production medium described in Example 6-3 and subjected to shaking culture at 33°C for 20 hours at 200 rpm.

After the cultivation was completed, the homoserine production ability (concentration) was measured using HPLC (Waters 2478). The experiment was repeated three times, and the average concentration value of homoserine as the analysis results is shown in Table 12 below.

**[TABLE 12]**

| Strain name | Homoserine (g/L) |
|---|---|
| KCCM12120P | 0.95 |
| CM10-0195(KCCM12120P_ *mqo*-*mdh*-*aspB*-*gdh*-*fumC-mqo*) | 1.19 |

As a result, as shown in Table 12, it was confirmed that the concentration of homoserine of the *Corynebacterium glutamicum* CM10-0196 strain in which the activities of malate:quinone oxidoreductase, malate dehydrogenase, aspartate transaminase, NADP-specific glutamate dehydrogenase, and fumarate hydratase were simultaneously enhanced was approximately 125% compared to the *Corynebacterium glutamicum* KCCM12120P strain, which is a homoserine-producing strain in which the activities of malate:quinone oxidoreductase, malate dehydrogenase, aspartate transaminase, NADP-specific glutamate dehydrogenase, and fumarate hydratase were non-enhanced.

From the above results, it was confirmed that the enhancement of the activity of fumarate hydratase and/or the simultaneous enhancement of the activities of malate:quinone oxidoreductase, malate dehydrogenase, aspartate transaminase, NADP-specific glutamate dehydrogenase, and fumarate hydratase increase the homoserine production ability of microorganisms of the genus *Corynebacterium*.

### Comparative Example 7. Preparation of a histidine-producing microorganism in which the activity of fumarate hydratase is not enhanced

As a control group for confirming the effect of increasing the productivity of glutamate-family amino acids according to the fumarate hydratase enhancement, the *Corynebacterium glutamicum* strain CA14-0114 in which genes of the histidine biosynthetic pathway were enhanced was prepared from wild-type *Corynebacterium glutamicum* ATCC13032. Specifically, in order to relieve the feedback inhibition of the HisG polypeptide, which is the first enzyme of the L-histidine biosynthetic pathway, the 233rd and 235th amino acids from the N-terminus of HisG were simultaneously substituted from glycine to histidine and from threonine to glutamine, respectively (SEQ ID NO: 74) (ACS Synth. Biol., 2014, 3 (1), pp 21-29). In addition, the start codon was substituted from GTG to ATG to enhance the activity of the hisE gene present in the same operon as hisG. Additionally, to enhance the L-histidine biosynthetic pathway, the promoters of the biosynthesis genes hisE, hisG, hisN, hisD, hisA, and hisB were replaced with strong promoters, and the corresponding pathway was enhanced by introducing additional copy numbers of the hisE(V1M)G(G233H/T235Q) operon and hisD gene.

### Comparative Example 7-1. Preparation of a histidine-producing strain with released feedback inhibition

In order to prepare a histidine-producing strain with relieved feedback inhibition, using the chromosomal DNA of the *Corynebacterium glutamicum* ATCC13032 strain as a template, the 'hisE(V1M)G(G233H/T235Q)' left DNA fragment was obtained by performing PCR using the primers of SEQ ID NOs: 86 and 87, and the 'hisE(V1M)G(G233H/T235Q)' right DNA fragment was obtained by performing PCR using the primers of SEQ ID NOs: 88 and 89. PfuUltra^{TM} high-fidelity DNA polymerase (Stratagene) was used as a polymerase for the PCR reaction. As PCR conditions, denaturation at 95°C for 30 seconds; annealing at 55°C for 30 seconds; and a polymerization reaction at 72°C for 1 minute were performed, and denaturation, annealing, and polymerization reaction under these conditions were repeated 28 times. Using the two amplified DNA fragments as templates, PCR was performed in the same manner as in Comparative Example 7-1 using the primers of SEQ ID NOs: 86 and 89, and as a result, the 'hisE(V1M)G(G233H/T235Q)' gene fragment was obtained. In addition, using the chromosomal DNA of the *Corynebacterium glutamicum* ATCC13032 strain as a template, PCR was performed using the primers of SEQ ID NOs: 90 and 91, and the upstream region of the hisE gene was obtained. The primer sequences used in Comparative Example 7-1 are shown in Table 13 below.

**[TABLE 13]**

| Sequence (5'-> 3') | SEQ ID NO |
|---|---|
| gaggagatcaaaacaATGAAGACATTTGAC | SEQ ID NO: 86 |
| ACACCAAGCTTGATGGATACCTCTACTGC A | SEQ ID NO: 87 |
| CAGTAGAGGTATCCATCAAGCTTGGTGTC | SEQ ID NO: 88 |
| ACTCTAGAGGATCCCCCTAGATGCGGGC | SEQ ID NO: 89 |
| TCGAGCTCGGTACCCACCGAACTCCTGAC AGAGT | SEQ ID NO: 90 |
| acatgaagcgccTCGGTACATTCTTCCACA | SEQ ID NO: 91 |
| AAGAATGTACCGAggcgcttcatgtcaaca | SEQ ID NO: 92 |
| CAAATGTCTTCATtgttttgatctcctcca | SEQ ID NO: 93 |
| ACTGCCTGGTACCACCAGA | SEQ ID NO: 94 |
| CTGCCTCTCACAAGTTGAAG | SEQ ID NO: 95 |

In order to replace with a strong promoter, PCR was performed in the same manner as in Comparative Example 7-1 using the Pspl13 promoter (SEQ ID NO: 9, Korean Registered Patent No. 10-1783170), which is a synthetic promoter, as a template and using the primers of SEQ ID NOs: 92 and 93.

After treating the pDC24 vector with the restriction enzyme SmaI, a recombinant plasmid was obtained by cloning the amplified upstream DNA fragment of the hisE gene, the Pspl13 promoter, and the 'hisE(V1M)G(G233H/T235Q)' gene fragment using the Gibson assembly (DG Gibson et al., NATURE METHODS, VOL.6 NO.5, MAY 2009, NEBuilder HiFi DNA Assembly Master Mix) method, and it was named pDC24ΔPn_hisEG::Pspl13_hisE(V1M)G(G233H/T235Q). Cloning was performed by mixing the Gibson assembly reagent and each gene fragment at the calculated molar amount and incubating at 50°C for 1 hour.

The prepared pDC24ΔPn_hisEG::Pspl13_hisE(V1M)G(G233H/T235Q) vector was transformed into *Corynebacterium glutamicum* ATCC13032 by electroporation, and then a strain in which hisE activity was enhanced was obtained by introducing mutations into the existing hisG gene through a second crossover process to relieve feedback inhibition and by replacing the start codon of the hisE gene. The corresponding genetic manipulation was confirmed through a PCR method and genome sequencing using the primers of SEQ ID NOs: 94 and 95, which can respectively amplify the external parts of the upstream and downstream regions of homologous recombination where the corresponding gene was inserted. The strain thus obtained was named CJ-HIS1.

### Comparative Example 7-2. Preparation of a histidine-producing strain in which the biosynthetic pathway is enhanced through promoter replacement

Subsequently, in order to replace the wild-type promoters of each gene with strong promoters to enhance the activity of the biosynthesis genes hisN, hisH, hisD, hisA, and hisB, a plasmid was prepared as follows.

Specifically, using *Corynebacterium glutamicum* ATCC13032 chromosomal DNA as a template, PCR was performed in the same manner as in Comparative Example 7-1 using the primers of SEQ ID NOs: 96 and 97, SEQ ID NOs: 98 and 99, SEQ ID NOs: 100 and 101, SEQ ID NOs: 102 and 103, and SEQ ID NOs: 104 and 105, and the upstream regions of the hisN, hisH, hisD, hisA, and hisB genes were obtained. In addition, using the chromosomal DNA of the *Corynebacterium glutamicum* ATCC13032 strain as a template and using the primers of SEQ ID NOs: 106 and 107, SEQ ID NOs: 108 and 109, SEQ ID NOs: 110 and 111, SEQ ID NOs: 112 and 113, and SEQ ID NOs: 114 and 115, the downstream regions of the hisN, hisH, hisD, hisA, and hisB genes were obtained.

In order to replace the native promoters of the *hisN, hisH,* and *hisD* genes with the Pcj7 promoter, which is a strong promoter, PCR was performed in the same manner as in Comparative Example 7-1 using the genomic DNA of *Corynebacterium* stationis as a template and using the primers of SEQ ID NOs: 116 and 117, SEQ ID NOs: 118 and 119, and SEQ ID NOs: 120 and 121.

In order to replace the native promoters of the *hisA* and *hisB* genes with the Pspl13 promoter, which is a strong promoter, PCR was performed in the same manner as in Comparative Example 7-1 using the Pspl13 promoter as a template and using the primers of SEQ ID NOs: 122 and 123, and SEQ ID NOs: 124 and 125.

After treating the pDC24 vector with the restriction enzyme SmaI, recombinant plasmids were obtained by cloning the amplified upstream DNA fragment, the Pcj7 promoter fragment, and the downstream DNA fragment for each of the *hisN, hisH*, and *hisD* genes, respectively, using the Gibson assembly method, which were named pDC24ΔPn::Pcj7_hisN, pDC24ΔPn:: Pcj7_hisH, and pDC24ΔPn:: Pcj7_hisD. In addition, after treating the pDC24 vector with the restriction enzyme SmaI, recombinant plasmids were obtained by cloning the amplified upstream DNA fragment, the Pspl13 promoter fragment, and the downstream DNA fragment for each of the *hisA* and *hisB* genes, respectively, using the Gibson assembly method, which were named pDC24ΔPn::Pspl13_hisA and pDC24ΔPn:: Pspl13_hisB. Gibson cloning was performed in the same manner as in Comparative Example 7-1.

The prepared pDC24ΔPn::Pcj7_hisN vector was transformed by electroporation into CJ-HIS1 prepared in Comparative Example 7-1, and then a strain in which the corresponding gene was enhanced was obtained by replacing the promoter of the existing hisN gene through a second crossover process. The corresponding genetic manipulation was confirmed through a PCR method and genome sequencing using the primers of SEQ ID NOs: 126 and 127, which can respectively amplify the external parts of the upstream and downstream regions of homologous recombination where the corresponding gene was inserted. The strain thus obtained was named CJ-HIS2.

Subsequently, the prepared pDC24ΔPn::Pcj7_hisH vector was transformed by electroporation into CJ-HIS2 prepared above, and then a strain in which the corresponding gene was enhanced was obtained by replacing the promoter of the existing hisH gene through a second crossover process. The corresponding genetic manipulation was confirmed through a PCR method and genome sequencing using the primers of SEQ ID NOs: 128 and 129, which can respectively amplify the external parts of the upstream and downstream regions of homologous recombination where the corresponding gene was inserted. The strain thus obtained was named CJ-HIS3.

Subsequently, the prepared pDC24ΔPn::Pcj7_hisD vector was transformed by electroporation into CJ-HIS3 prepared above, and then a strain in which the corresponding gene was enhanced was obtained by replacing the promoter of the existing hisD gene through a second crossover process. The corresponding genetic manipulation was confirmed through a PCR method and genome sequencing using the primers of SEQ ID NOs: 130 and 131. The strain thus obtained was named CJ-HIS4.

Subsequently, the prepared pDC24ΔPn::Pspl13_hisA vector was transformed by electroporation into CJ-HIS4 prepared above, and then a strain having the corresponding gene enhanced was obtained by replacing the promoter of the existing hisA gene through a second crossover process. The corresponding genetic manipulation was confirmed through a PCR method and genome sequencing using the primers of SEQ ID NOs: 132 and 133. The strain thus obtained was named CJ-HIS5.

Subsequently, the prepared pDC24ΔPn::Pspl13_hisB vector was transformed by electroporation into the CJ-HIS5 prepared above, and then a strain in which the corresponding gene was enhanced was obtained by replacing the promoter of the existing hisB gene through the second crossover process. The corresponding genetic manipulation was confirmed through a PCR method and genome sequencing using the primers of SEQ ID NOs: 134 and 135. The strain thus obtained was named CJ-HIS6.

The primer sequences used in Comparative Example 7-2 are shown in Table 14 below.

**[TABLE 14]**

| Sequence (5'-> 3') | SEQ ID NO |
|---|---|
| TCGAGCTCGGTACCCATTGGTGCTCGGCGC | SEQ ID NO: 96 |
| tgggatgtttctGTGTTGTTAGTCTAGTG | SEQ ID NO: 97 |
| TCGAGCTCGGTACCCAACCAAGTTTAGATGCGCC | SEQ ID NO: 98 |
| gctgggatgtttctGCCGATAGTTTATGTCA | SEQ ID NO: 99 |
| TCGAGCTCGGTACCCGGTGACAGCTCGCGCCGCAT | SEQ ID NO: 100 |
| gcgctgggatgtttctGGCGAAAAGTTCTCCC | SEQ ID NO: 101 |
| TCGAGCTCGGTACCCTTGATGCCTGCATGAAGG | SEQ ID NO: 102 |
| tgacatgaagcgccGAATATTGATCCTATCT | SEQ ID NO: 103 |
| TTCGAGCTCGGTACCCACCTTCAGCAACCACTC | SEQ ID NO: 104 |
| tgacatgaagcgccGAAAAATTCTTCTCT | SEQ ID NO: 105 |
| aaaggaaacactcATGAGCAAATATGCAGACG | SEQ ID NO: 106 |
| CTAGAGGATCCCCCAGCCGGAGAGGGA | SEQ ID NO: 107 |
| aaaggaaacactcATGACCAAAACTGTCGC | SEQ ID NO: 108 |
| CTAGAGGATCCCCACCTCTGGAGGCGTGGTC | SEQ ID NO: 109 |
| cgaaaggaaacactcATGTTGAATGTCACTGACC | SEQ ID NO: 110 |
| CTAGAGGATCCCCCCGTGCTCAGCCTGAGGAG | SEQ ID NO: 111 |
| ggagatcaaaacaATGACCTTCACTATTCTTCC | SEQ ID NO: 112 |
| CTAGAGGATCCCCACGAAACGTGCACAACCTT | SEQ ID NO: 113 |
| gagatcaaaacaATGACTGTCGCACCA | SEQ ID NO: 114 |
| CTAGAGGATCCCCGGGTCGCGGCCGTAGTGGC | SEQ ID NO: 115 |
| ACTAGACTAACAACACagaaacatcccagcgc | SEQ ID NO: 116 |
| TCTGCATATTTGCTCATgagtgtttccttt | SEQ ID NO: 117 |
| ACATAAACTATCGGCagaaacatcccagcgcta | SEQ ID NO: 118 |
| GACAGTTTTGGTCATgagtgtttcctttcg | SEQ ID NO: 119 |
| GAGAACTTTTCGCCagaaacatcccagcgct | SEQ ID NO: 120 |
| AGTGACATTCAACATgagtgtttcctttcg | SEQ ID NO: 121 |
| AGGATCAATATTCggcgcttcatgtcaac | SEQ ID NO: 122 |
| GAATAGTGAAGGTCATtgttttgatctcct | SEQ ID NO: 123 |
| GAGAAGAATTTTTCggcgcttcatgtcaa | SEQ ID NO: 124 |
| TGGTGCGACAGTCATtgttttgatctcct | SEQ ID NO: 125 |
| GAGCATGCATCAAAG | SEQ ID NO: 126 |
| AGAAATTTGATCCTTATAA | SEQ ID NO: 127 |
| TTGAGAGATGCTTATCG | SEQ ID NO: 128 |
| CACTTCAGTGCGGATTCCAA | SEQ ID NO: 129 |
| AGCGGGTTTAATTCAGG | SEQ ID NO: 130 |
| GTGGGTAAGGGTTTTCGT | SEQ ID NO: 131 |
| CACGAAAATGATCGTTTTG | SEQ ID NO: 132 |
| TATGGGATTCGATGGCCA | SEQ ID NO: 133 |
| TGTGGGAATCGCTGGGCAC | SEQ ID NO: 134 |
| CGGTCGCCCGCATCTG | SEQ ID NO: 135 |

### Comparative Example 7-3. Preparation of a histidine-producing strain in which the biosynthetic pathway is enhanced through additional gene insertion

Subsequently, in order to additionally insert the hisE(V1M)G(G233H/T235Q) operon and the hisD gene, NCgl1021, which is known as a gene encoding a transposon in *Corynebacterium glutamicum*, was used as an insertion site. Specifically, in order to prepare an NCgl1021 (SEQ ID NO: 70) deletion and target gene insertion vector, PCR was performed in the same manner as in Comparative Example 7-1 using the chromosome of ATCC13032 as a template and using the primer pairs of SEQ ID NOs: 136 and 137, and SEQ ID NOs: 138 and 139. Using the vector pDC24ΔPn_hisEG::Pspl13_hisE(V1M)G(G233H/T235Q) prepared in Comparative Example 7-1 as a template, PCR was performed in the same manner as in Comparative Example 7-1 using the primers of SEQ ID NOs: 140 and 141, and the 'Pspl13_hisE(V1M)G(G233H/T235Q)' gene fragment was obtained.

In addition, using the vector pDC24ΔPn::Pcj7_hisD prepared in Comparative Example 7-2 as the template, PCR was performed in the same manner as in Example 1 using the primers of SEQ ID NOs: 142 and 143, and the 'Pcj7_hisD' gene fragment was obtained.

After treating the pDC24 vector with the restriction enzyme SmaI, a recombinant plasmid was obtained by cloning the the left homologous arm region of NCgl1021, the right homologous arm region of NCgl1021,'Pspl13_hisE(V1M)G(G233H/T235Q)', and 'Pcj7_hisD' using the Gibson assembly method, and it was named 'pDC24ΔNCgl1021::Pspl13_hisE(V1M)G(G233H/T235Q)-Pcj7_hisD'. Gibson cloning was performed in the same manner as in Comparative Example 7-1. The prepared 'pDC24ΔNCgl1021::Pspl13_hisE(V1M)G(G233H/T235Q)-Pcj7_hisD' vector was transformed by electroporation into CJ-HIS6 prepared in Comparative Example 7-2, and then a strain in which the histidine biosynthetic pathway was enhanced through additional gene insertion was obtained through a second crossover process. The corresponding genetic manipulation was confirmed through a PCR method and genome sequencing using the primers of SEQ ID NOs: 144 and 145, which can respectively amplify the homologous recombination upstream region and the external part of the downstream region where the corresponding gene was inserted. The strain thus obtained was named CA14-0114.

The primer sequences used in Comparative Example 7-3 are shown in Table 15 below.

**[TABLE 15]**

| Sequence (5'-> 3') | SEQ ID NO |
|---|---|
| TTCGAGCTCGGTACCCATGAAGTCTACCG GC | SEQ ID NO: 136 |
| gacatgaagcgccGACATCTAATAACCGGG | SEQ ID NO: 137 |
| CCGACGAGGCCTAAGAACTCATTCCTTCTG CT | SEQ ID NO: 138 |
| CTCTAGAGGATCCCCTTAGAGTGCATTGAT C | SEQ ID NO: 139 |
| CCGGTTATTAGATGTCggcgcttcatgtca | SEQ ID NO: 140 |
| ggatgtttctCTAGATGCGGGCGAT | SEQ ID NO: 141 |
| GCCCGCATCTAGagaaacatcccagcgct | SEQ ID NO: 142 |
| AGAAGGAATGAGTTCTTAGGCCTCGTCGG | SEQ ID NO: 143 |
| CTTTCAGCTTTCCCTCCCG | SEQ ID NO: 144 |
| GCTGTACTTTTAGTACA | SEQ ID NO: 145 |

### Example 7. Preparation of a histidine-producing microorganism having enhanced fumarate hydratase activity and evaluation of histidine production ability

### Example 7-1. Preparation of histidine-producing microorganism having enhanced fumarate hydratase activity

In order to confirm the effect of enhancement of activity of fumarate hydratase on histidine production in a *Corynebacterium glutamicum* strain having histidine production ability, the pDC24-ΔNCgl1490:Pspl13-fumC plasmid prepared in Example 5-1 was transformed into the *Corynebacterium glutamicum* CA14-0114 strain, which is a histidine-producing strain prepared in Comparative Example 7-3, by the electroporation to obtain a transformant. Then, PCR was performed on the transformant using the primer pair of SEQ ID NOs: 58 and 62 to confirm that fumarate hydratase was introduced into the chromosome in a form wherein its expression was enhanced by Pspl13. The PCR reaction was performed by repeating 30 cycles of: denaturation at 95°C for 30 seconds; annealing at 55°C for 30 seconds; and extension at 72°C for 2 minutes. The transformant was named *Corynebacterium glutamicum* CA14-0114 ΔNCgl1490-91::Pspl13-fumC strain.

### Example 7-2. Evaluation of the increase in histidine production ability of a histidine-producing microorganism having enhanced fumarate hydratase activity

In order to confirm the increase in the histidine production ability of a histidine-producing microorganism having enhanced fumarate hydratase activity, the strains were cultured in the following manner to analyze the histidine concentration in the culture broth.

Specifically, the control group *Corynebacterium glutamicum* CA14-0114 strain and the *Corynebacterium glutamicum* CA14-0114 ΔNCgl1490-91::Pspl13-fumC strain prepared in Comparative Example 7-3 were each inoculated into a 250 ml corner-baffle flask containing 25 ml of the following seed medium and subjected to shaking culture at 33°C for 20 hours at 200 rpm. Then, 1 ml of the seed culture broth was inoculated into a 250 ml corner-baffle flask containing 25 ml of the production medium and subjected to shaking culture at 30°C for 24 hours at 200 rpm.

### <Seed medium (pH 7.0)>

Glucose 5%, bacto peptone 1%, sodium chloride 0.25%, yeast extract 1%, urea 0.4%

### <Histidine production medium (pH 7.2)>

glucose 5%, ammonium sulfate 2%, monopotassium phosphate 0.1%, magnesium sulfate heptahydrate 0.05%, CSL (corn steep liquor) 2.0%, biotin 200 µg/L, calcium carbonate 30 g/L (based on 1 liter of distilled water)

After the cultivation was completed, histidine production ability (concentration) was measured using HPLC (Waters 2478). The experiment was repeated three times, and the average concentration of histidine as the analysis results is shown in Table 16 below.

**[TABLE 16]**

| Strain name | Histidine (g/L) |
|---|---|
| CA14-0114 | 4.6 |
| CA14-0114Δ*NCgl1490*-91::Pspl13-*fumC* | 4.7 |

As a result, as shown in Table 16, it was confirmed that the histidine concentration in the culture broth of the *Corynebacterium glutamicum* CA14-0114 ΔNCgl1490-91::Pspl13-fumC strain, in which the activity of fumarate hydratase was enhanced, was approximately 102% compared to the *Corynebacterium glutamicum* CA14-0114 strain, which is a histidine-producing strain in which the activity of fumarate hydratase was non-enhanced.

### Example 7-3. Preparation of a histidine-producing microorganism in which the activities of malate:quinone oxidoreductase, malate dehydrogenase, aspartate transaminase, NADP-specific glutamate dehydrogenase, and fumarate hydratase are simultaneously enhanced

In order to prepare a histidine-producing microorganism in which the activities of malate:quinone oxidoreductase, malate dehydrogenase, aspartate transaminase, NADP-specific glutamate dehydrogenase, and fumarate hydratase are all enhanced, the pDC24Δmqo-mdh-aspB-gdh-fumC-mqo plasmid prepared in Example 3 was transformed into *Corynebacterium glutamicum* CA14-0114, which is a histidine-producing strain, to obtain a recombinant strain (van der Rest et al., Appl Microbiol Biotechnol 52:541-545, 1999). PCR was performed on the obtained recombinant strain using the primer pair of SEQ ID NOs: 54 and 55 to confirm that the mqo-mdh-aspB-gdh-fumC-mqo complex was introduced into the chromosome. The PCR reaction was performed by repeating 30 cycles of: denaturation at 95°C for 30 seconds; annealing at 55°C for 30 seconds; and extension at 72°C for 2 minutes. The transformant was named *Corynebacterium glutamicum* CA14-0114_mqo-mdh-aspB-gdh-fumC-mqo strain.

### Example 7-4. Evaluation of increase in histidine production ability of a histidine-producing microorganism in which the activities of malate:quinone oxidoreductase, malate dehydrogenase, aspartate transaminase, NADP-specific glutamate dehydrogenase, and fumarate hydratase are simultaneously enhanced.

In order to confirm the increase in the histidine production ability of a histidine-producing microorganism in which the activities of malate:quinone oxidoreductase, malate dehydrogenase, aspartate transaminase, NADP-specific glutamate dehydrogenase, and fumarate hydratase are simultaneously enhanced, the strains were cultured in the following manner to analyze the histidine concentration in the culture broth.

Specifically, the control group *Corynebacterium glutamicum* CA14-0114 strain and the *Corynebacterium glutamicum* CA14-0114_mqo-mdh-aspB-gdh-fumC-mqo strain prepared in Example 7-3 were each inoculated into a 250 ml corner-baffle flask containing 25 ml of the seed medium described in Example 7-2 and subjected to shaking culture at 33°C for 20 hours at 200 rpm. Then, 1 ml of the seed culture broth was inoculated into a 250 ml corner-baffle flask containing 25 ml of the production medium described in Example 7-2 and subjected to shaking culture at 30°C for 24 hours at 200 rpm.

After the cultivation was completed, the histidine production ability (concentration) was measured using HPLC (Waters 2478). The experiment was repeated three times, and the average concentration of histidine as the analysis results is shown in Table 17 below.

**[TABLE 17]**

| Strain name | Histidine (g/L) |
|---|---|
| CA14-0114 | 4.6 |
| CA14-0114_*mqo*-*mdh*-*aspB*-*gdh*-*fumC*-*mqo*) | 5.2 |

As a result, as shown in Table 17, it was confirmed that the histidine concentration in the culture broth of the *Corynebacterium glutamicum* CA14-0114_mqo-mdh-aspB-gdh-fumC-mqo strain, in which the activities of malate:quinone oxidoreductase, malate dehydrogenase, aspartate transaminase, NADP-specific glutamate dehydrogenase, and fumarate hydratase were simultaneously enhanced, was approximately 113% compared to the *Corynebacterium glutamicum* CA14-0114 strain, a histidine-producing strain in which the activities of malate:quinone oxidoreductase, malate dehydrogenase, aspartate transaminase, NADP-specific glutamate dehydrogenase, and fumarate hydratase were not enhanced.

From the above results, it was confirmed that the enhancement of the activity of fumarate hydratase and/or the simultaneous enhancement of the activities of malate:quinone oxidoreductase activity, malate dehydrogenase activity, aspartate transaminase activity, NADP-specific glutamate dehydrogenase activity, and fumarate hydratase increase the histidine production ability of microorganisms of the genus *Corynebacterium*.

From the above description, those person skilled in the art to which the present disclosure pertains will understand that the present disclosure may be practiced in other specific forms without changing its technical spirit or essential features. In this regard, it should be understood that the examples described above are illustrative in all respects and not restrictive. The scope of the present disclosure should be interpreted such that all modifications or variations derived from the meaning and scope of the following claims and their equivalent concepts are included in the scope of the present disclosure, rather than being limited by the above detailed description.

## Claims

1. A microorganism of the genus *Corynebacterium* producing an L-amino acid, having an enhanced activity of fumarate hydratase.

2. The microorganism of the genus *Corynebacterium* according to claim 1, wherein the fumarate hydratase comprises an amino acid sequence having 90% or more sequence identity with the amino acid sequence of SEQ ID NO: 1.

3. The microorganism of the genus *Corynebacterium* according to claim 1, wherein the fumarate hydratase is encoded by a polynucleotide comprising a nucleic acid sequence having 90% or more sequence identity with the nucleic acid sequence of SEQ ID NO: 2 or SEQ ID NO: 41.

4. The microorganism of the genus *Corynebacterium* according to claim 1, wherein the microorganism of the genus *Corynebacterium* additionally has enhanced activities of malate:quinone oxidoreductase, malate dehydrogenase, aspartate transaminase, and NADP-specific glutamate dehydrogenase.

5. The microorganism of the genus *Corynebacterium* according to claim 4, wherein the malate:quinone oxidoreductase consists of the amino acid sequence of SEQ ID NO: 30, the malate dehydrogenase consists of the amino acid sequence of SEQ ID NO: 34, the aspartate transaminase consists of the amino acid sequence of SEQ ID NO: 36, and the NADP-specific glutamate dehydrogenase consists of the amino acid sequence of SEQ ID NO: 38.

6. The microorganism of the genus *Corynebacterium* according to claim 1, wherein the microorganism is *Corynebacterium glutamicum*.

7. The microorganism of the genus *Corynebacterium* according to claim 1, wherein the microorganism has increased L-amino acid production ability compared to a non-modified microorganism of the same species in which the activity of fumarate hydratase is not enhanced.

8. The microorganism of the genus *Corynebacterium* according to claim 1, wherein the L-amino acid is selected from the group consisting of glutamate-family amino acids, homoserine-family amino acids, and L-histidine.

9. The microorganism of the genus *Corynebacterium* according to claim 8, wherein the glutamate-family amino acids are selected from the group consisting of L-arginine, L-citrulline, and L-ornithine.

10. The microorganism of the genus *Corynebacterium* according to claim 8, wherein the homoserine-family amino acids are selected from the group consisting of O-acetyl homoserine and L-homoserine.

11. A method for producing an L-amino acid, comprising culturing the microorganism of the genus *Corynebacterium* of any one of claims 1 to 10 in a medium; and recovering the L-amino acid from the cultured microorganism, the medium, or both.

12. The method for producing an L-amino acid according to claim 11, wherein the microorganism of the genus *Corynebacterium* is *Corynebacterium glutamicum*.

13. The method for producing an L-amino acid according to claim 11, wherein the L-amino acid is selected from the group consisting of glutamate-family amino acids, homoserine-family amino acids, and L-histidine.

14. The method for producing an L-amino acid according to claim 13, wherein the glutamate-family amino acids are selected from the group consisting of L-arginine, L-citrulline, and L-ornithine.

15. The method for producing an L-amino acid according to claim 13, wherein the homoserine-family amino acids are selected from the group consisting of O-acetyl homoserine and L-homoserine.

16. A use of the microorganism of the genus *Corynebacterium* of claim 1 for producing an L-amino acid.
